# EUROPEAN PATENT APPLICATION

(11) **EP 4 830 695 A1**
(43) Date of publication of application: **28.10.2026**
(21) Application number: 24907203.4
(22) Date of filing: 06.12.2024
(51) Int. Cl.: A61K 8/891, A61K 8/19, A61K 8/29, A61K 8/31, A61K 8/34, A61K 8/37, A61K 8/92, A61Q 1/00, A61Q 1/06, A61Q 1/10, A61Q 17/04, A61Q 19/00

(54) **COSMETIC**

(30) Priority: 18.12.2023 JP 2023212897
(71) Applicant: SHIN-ETSU CHEMICAL CO., LTD., Tokyo 100-0005 (JP)
(72) Inventor: KAMEI Masanao, Annaka-shi, Gunma 379-0224 (JP)
(74) Representative: Mewburn Ellis LLP
(86) International application number: PCT/JP2024/043244
(87) International publication number: WO 2025/134817

(57) **Abstract**

Provided is a cosmetic containing a carboxylic acid-modified silicone, said cosmetic comprising:
(A) 0.1-10% by mass of a carboxylic acid-modified silicone represented by formula (1) and having a weight-average molecular weight of 1,000-5,000;
(B) 1-90% by mass of an oil; and
(C) 2-95% by mass of a powder.
The cosmetic has excellent powder dispersibility and dispersion stability, good spreadability and skin compatibility, and excellent stability over time.

## Description

### TECHNICAL FIELD

The present invention relates to a cosmetic.

### BACKGROUND ART

Cosmetics, such as makeups, sunscreens, concealers, and makeup bases, contain powders. It is known that powder aggregation dulls the color and causes the finish to look less beautiful, or the aggregates produce a resistant feel to the touch, and, in the case of titanium dioxide and zinc oxide in UV scattering agents, reduce the UV protection effect.

On the other hand, oils that are blended into cosmetics are selected from, for example, hydrocarbon oils, higher fatty acids, higher alcohols, esters, polar oils, such as organic UV absorbers, and silicone oils, depending on the purpose. In particular, silicone oils, which are characterized by their light feel, are frequently used. Thus, many powder dispersant techniques using modified silicones that are compatible with silicone oils have been developed. According to such methods proposed so far, for example, a polyether-modified silicone is used as a dispersant (Patent Document 1), and a polyglycerin-modified silicone is used (Patent Document 2). The above oils are often used in combination. The fact that their molecular weights and molecular polarities are different makes it necessary to determine, for example, the compatibility of the oils to be combined, and the feel before they are used in cosmetics. The techniques proposed to improve the compatibility with oils other than silicones include a silicone compound that has a long-chain alkyl group and a polyoxyalkylene group (Patent Document 3), a silicone compound that has a long-chain alkyl group and a polyhydric alcohol (Patent Document 4), and a silicone compound that has a long-chain alkyl group, a glycerin group, and a branched-chain silicone group (Patent Document 5). Because polyoxyalkylene groups, polyhydric alcohols, and glycerin groups are highly hydrophilic and thus the compounds also act as surfactants, unfortunately, the use of the above compounds in emulsion cosmetics may cause deterioration in dispersion stability and emulsion stability depending on the types of other surfactants used in combination therewith.

Furthermore, a carboxylic acid-modified silicone compound (Patent Document 6) and a carboxylic acid-modified silicone compound with a molecular weight of 800 or less (Patent Document 7) have been proposed. Carboxylic acids are good dispersants because they have weak emulsifying properties and exhibit good adsorption stability with respect to powders. When, however, powders are added in large amounts, the carboxylic acid-modified silicones of the related art cannot reduce the cohesive force among the particles and the dispersion viscosity is increased. In view of the fact that dispersants prevent a powder from reaggregation by adsorbing to the powder surface and forming a strong adsorption layer, it is important how strongly the organic groups in the dispersants can adsorb to powders. Dispersants that offer higher dispersion stability are thus desired.

### PRIOR ART DOCUMENTS

### PATENT DOCUMENTS

Patent Document 1: JP-A H10-167946
Patent Document 2: JP-A H10-316536
Patent Document 3: JP-A S61-090732
Patent Document 4: JP-A 2002-179798
Patent Document 5: WO 2022/172816
Patent Document 6: JP-A 2002-080771
Patent Document 7: JP-A 2013-177370

### SUMMARY OF INVENTION

### TECHNICAL PROBLEM

An object of the present invention is to provide a cosmetic that contains a carboxylic acid-modified silicone and that has excellent powder dispersibility and dispersion stability, exhibits good spreadability, is well-fitting to the skin, and excels in stability over time.

### SOLUTION TO PROBLEM

The present inventor carried out extensive studies directed to achieving the above object. As a result, the present inventor has found that a carboxylic acid-modified silicone in which a carboxyl group is bonded to a silicon atom via an ester bond can easily adsorb to the surface of powder particles and can form a strong adsorption layer to prevent the powder particles from aggregation and allow the powder to attain excellent dispersibility and dispersion stability. The present inventor has found that a cosmetic containing this silicone exhibits good spreadability, is well-fitting to the skin, and excels in stability over time. The present invention has been thus completed.

Specifically, the present invention provides the following cosmetics:
1. A cosmetic including:
   (A) 0.1 to 10% by mass of a carboxylic acid-modified silicone with a weight average molecular weight of 1,000 to 5,000 represented by formula (1) below:
      [Chem. 1]

      [R¹₂SiO_{2/2}]ₐ[R¹R²SiO_{2/2}]_{b}[R¹R³SiO_{2/2}]_{c}[R¹SiO_{3/2}]_{d}]SiO_{4/2}]ₑ [R¹₃SiO_{1/2}]_{f} [R¹R²SiO_{1/2}]_{g} (1)

      [wherein R¹ independently at each occurrence is a monovalent organic group selected from C₁-C₂₀ alkyl groups, C₆-C₁₅ aryl groups, C₇-C₁₅ aralkyl groups, and -Q-O-R⁴ (Q is a C₂-C₄ alkylene group, and R⁴ is a C₃-C₃₀ alkyl group), R² independently at each occurrence is a carboxylic acid group represented by formula (2) below: 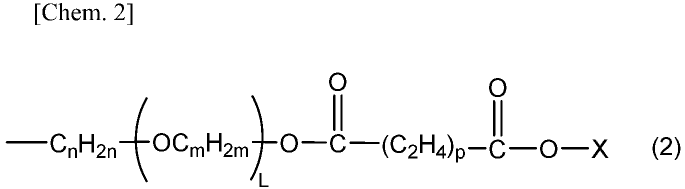 (wherein X denotes a hydrogen atom or a monovalent cation, n is 2 to 4, m is 2 to 4, L is 0 to 20, and p is 2 to 6),
      R³ is a group represented by formula (3) or (4) below: 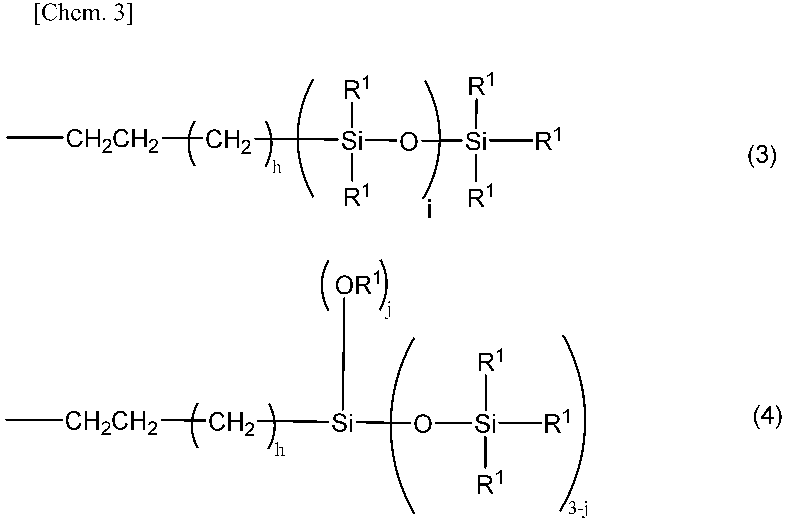 (wherein R¹ independently at each occurrence is the same as above, h is 0 to 5, i is 0 to 80, and j is 0 to 3),
      a is 0 to 20, b is 0 to 5, c is 0 to 3, d is 0 or greater, e is 0 or greater, f and g are numbers satisfying f + g = 2 + d + e × 2, and b and g are not 0 at the same time];
   (B) 1 to 90% by mass of an oil; and
   (C) 2 to 95% by mass of a powder.
2. The cosmetic according to 1, further including (D) 0.1 to 30% by mass of an organic UV absorber.
3. The cosmetic according to 1 or 2, wherein the oil (B) is one or more selected from silicone oils, hydrocarbon oils, ester oils, glyceride oils, natural animal and plant oils and fats, semi-synthetic oils and fats, and higher alcohols.
4. The cosmetic according to any one of 1 to 3, wherein the powder (C) is one or more selected from titanium dioxide, zinc oxide, and colored pigments.
5. The cosmetic according to any one of 1 to 4, wherein the cosmetic further includes a surfactant (E) and water, and is an emulsion.

### ADVANTAGEOUS EFFECTS OF INVENTION

The cosmetic according to the present invention, by including a carboxylic acid-modified silicone offering excellent powder dispersibility and dispersion stability, exhibits good spreadability, is well-fitting to the skin, and excels in stability over time.

### DESCRIPTION OF EMBODIMENTS

The present invention will be described in detail below. In the present invention, the name of ingredients is sometimes expressed by the Japanese labeling name or the International Nomenclature of Cosmetic Ingredients (INCI). When the Japanese labeling name is identical with the INCI, sometimes either one is omitted.

### [Carboxylic acid-modified silicones (A)]

A carboxylic acid-modified silicone (A) is represented by formula (1) below. Such carboxylic acid-modified silicones may be used singly, or two or more may be used in combination.
[Chem. 4]

[R¹₂SiO_{2/2}]ₐ[R¹R²SiO_{2/2}]_{b}[R¹R³SiO_{2/2}]_{c}[R¹SiO_{3/2}]_{d}[SiO_{4/2}]ₑ [R¹₃SiO_{1/2}]_{f} [R¹R²SiO_{1/2}]_{g} (1)

[wherein R¹ independently at each occurrence is a monovalent organic group selected from C₁-C₂₀ alkyl groups, C₆-C₁₅ aryl groups, C₇-C₁₅ aralkyl groups, and -O-R⁴ (Q is a C₂-C₄ alkylene group, and R⁴ is a C₃-C₃₀ alkyl group), R² independently at each occurrence is a carboxylic acid group represented by formula (2) below: 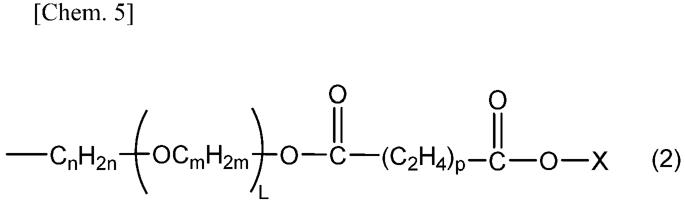 (wherein X denotes a hydrogen atom or a monovalent cation, n is 2 to 4, m is 2 to 4, L is 0 to 20, and p is 2 to 6),
R³ is a group represented by formula (3) or (4) below: 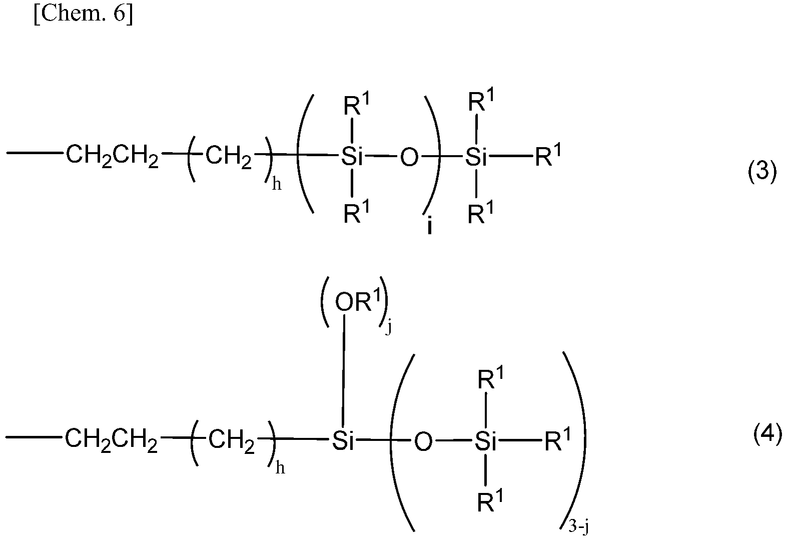 (wherein R¹ independently at each occurrence is the same as above, h is 0 to 5, i is 0 to 80, and j is 0 to 3),
a is 0 to 20, b is 0 to 5, c is 0 to 3, d is 0 or greater, e is 0 or greater, f and g are numbers satisfying f + g = 2 + d + e × 2, and b and g are not 0 at the same time.]

R¹ independently at each occurrence is a monovalent organic group selected from C₁-C₂₀ alkyl groups, C₆-C₁₅ aryl groups, C₇-C₁₅ aralkyl groups, and -Q-O-R⁴ (Q is a C₂-C₄ alkylene group, and R⁴ is a C₃-C₃₀ alkyl group). Specific examples include linear or branched alkyl groups, such as methyl group, ethyl group, propyl group, butyl group, hexyl group, octyl group, decyl group, dodecyl group, tetradecyl group, and hexadecyl group; cycloalkyl groups, such as cyclopentyl group and cyclohexyl group; aryl groups, such as phenyl group and tolyl group; aralkyl groups, such as benzyl group; -C₂H₄OC₁₁H₂₃, -C₂H₄OC₁₂H₂₅, -C₃H₆OC₁₆H₃₃, -C₃H₆OC₁₈H₃₇, and -C₃H₄OC₂₂H₄₅. In particular, methyl group is preferable. When the oil that is used is, for example, a hydrocarbon oil, a higher fatty acid, a higher alcohol, an ester, or a polar oil, such as a UV absorber, dodecyl group, hexadecyl group, and -C₃H₆OC₁₈C₃₇ are also preferable.

R² independently at each occurrence is a carboxyl group that is bonded via an ester bond as shown by formula (2) below: 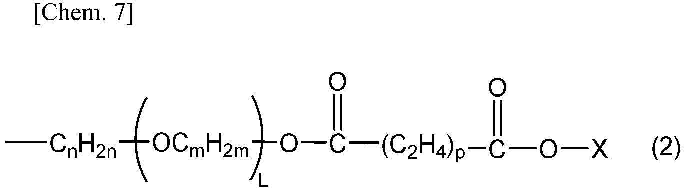 (wherein X denotes a hydrogen atom or a monovalent cation, n is 2 to 4, m is 2 to 4, L is 0 to 20, and p is 2 to 6).

X is a hydrogen atom or a monovalent cation. Specific examples of the monovalent cations include alkali metal ions, such as lithium, sodium, and potassium, and salts, such as ammonium and alkylamines. X is preferably a hydrogen atom, sodium, or potassium. In the monovalent organic group containing a carboxyl group, n is 2 to 4, m is 2 to 4, L is 0 to 20, and p is 2 to 6. As a result of the carboxyl group being bonded via an ester bond, the silicone can easily adsorb to the surface of powder particles and can form a strong adsorption layer to prevent the powder particles from aggregation, thus enhancing the dispersibility and the dispersion stability of the powder. Specific examples include the following formulas (5) to (7): 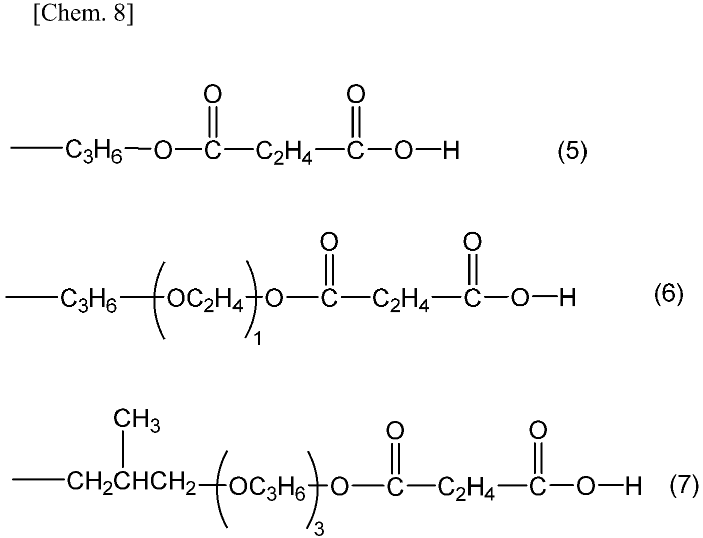 R³ is a group represented by formula (3) or (4) below: 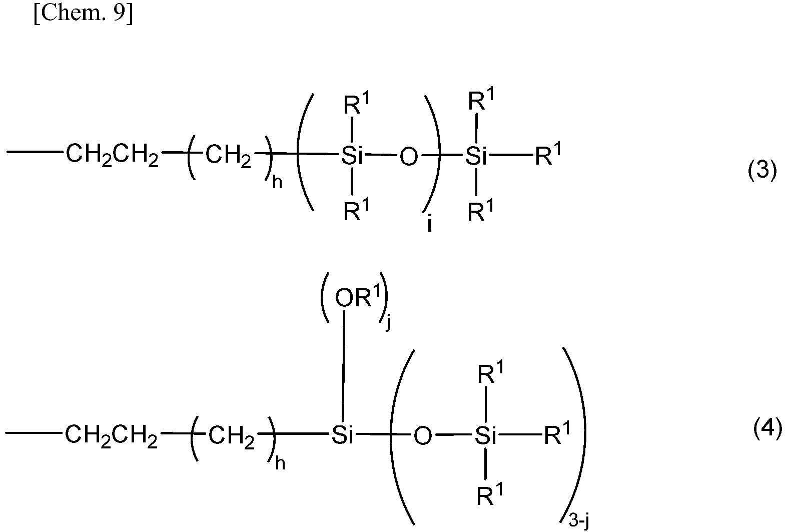 (wherein R¹ independently at each occurrence is the same as above, h is 0 to 5, i is 0 to 80, and j is 0 to 3).

R¹ independently at each occurrence is the same as above. The letter h is 0 to 5. The letter h is 0 when the corresponding moiety is obtained by the addition reaction between a vinylsiloxy group and a hydrosilyl group. The letter i is 0 to 80, and preferably 1 to 10. The letter i is 0 to 80, and preferably 1 to 10. If i is greater than 80, the dispersant is less adsorptive to powders and causes deterioration in dispersion stability. The letter j is 0 to 3, and preferably 0 or 1.

The letter a is 0 to 20, b is 0 to 5, c is 0 to 3, d is 0 or greater, e is 0 or greater, f and g are numbers satisfying f + g = 2 + d + e × 2, and b and g are not 0 at the same time. If a is greater than 20 or c is greater than 3, the dispersant is less adsorptive to powders and causes deterioration in dispersion stability. If b is greater than 5, the polarity is so high that the viscosity of the dispersant itself is increased and the dispersion stability is deteriorated. The compatibility differs depending on the type of the oil that is used, and thus preferred values of these letters are variable. It is, however, preferable that a be 0 to 10, b be 1 to 3, c be 0 to 3, d be 0 to 3, e be 0 to 3, and f + g be 2 to 11.

The weight average molecular weight of the carboxylic acid-modified silicone (A) is 1,000 to 5,000. From the point of view of the balance between the viscosity of the dispersant itself and the compatibility with oils, the weight average molecular weight is preferably 1,000 to 3,000. If the weight average molecular weight is less than 1,000, the dispersion stability is deteriorated over time.

In the present invention, the molecular weight indicates a value of weight average molecular weight determined by gel permeation chromatography (GPC) analysis using polystyrene standards under the following conditions.

### [Measurement conditions]

| | |
|---|---|
| Developing solvent: | Tetrahydrofuran (THF) |
| Flow rate: | 0.6 mL/min |
| Detector: | Differential refractive index detector (RI) |
| Columns: | TSK Guardcolumn Super H-H |
| | TSKgel SuperHM-N (6.0 mm I.D. × 15 cm × 1) |
| | TSKgel SuperH2500 (6.0 mm I.D. × 15 cm × 1) |
| | (all manufactured by Tosoh Corporation) |
| Column temperature: | 40°C |
| Sample injection volume: | 50 µL (0.3 % by mass THF solution) |

The amount of the component (A) in the cosmetic is 0.1 to 10% by mass, and preferably 0.5 to 5% by mass. If the amount is less than 0.1% by mass, the dispersion stability of a powder is deteriorated. If the amount is more than 10% by mass, the dispersant will produce a sticky feel.

The carboxylic acid-modified silicone (A) may be prepared by a common method. The carboxylic acid-modified silicone of the present invention may be produced using a known technique. For example, the carboxylic acid-modified silicone may be obtained by the addition reaction of a terminal unsaturated group-containing hydroxy compound onto a hydrosilyl-containing organopolysiloxane, followed by the ring-opening reaction with succinic anhydride. More specifically, the production method may include a step of reacting an organohydrogenpolysiloxane with a glycol having an unsaturated group in the presence of an addition reaction catalyst to produce a hydroxyl-containing dimethylpolysiloxane, and a step of performing the ring-opening reaction of an acid anhydride compound, such as succinic anhydride, onto the product obtained above.

### [Oils (B)]

Examples of the oils (B) include silicone oils, hydrocarbon oils, ester oils, glyceride oils, natural animal and plant oils and fats, semi-synthetic oils and fats, and higher alcohols. Any oils that are usually used in cosmetics, pharmaceuticals, and the like may be used without limitation. The oils may be used singly, or two or more may be used in combination.

Examples of the silicone oils include Cyclotetrasiloxane (INCI), Cyclopentasiloxane (INCI), Cyclohexasiloxane (INCI), Dimethicone (INCI), Methyl Trimethicone (INCI), tetrakis(trimethylsiloxy)silane, Caprylyl Methicone (INCI), Phenyl Trimethicone (INCI), Diphenylsiloxy Phenyl Trimethicone (INCI), Diphenyl Dimethicone (INCI), trifluoromethylalkyl (C1-4) dimethicone, Amodimethicone (INCI), Aminopropyl Dimethicone (INCI), and PCA Dimethicone (INCI).

Examples of the hydrocarbon oils include linear hydrocarbon oils, branched hydrocarbon oils, and volatile hydrocarbon oils. Specific examples include Olefin Oligomers (INCI), isoparaffins, such as (C13,C14) Isoparaffin (INCI), and alkanes, such as Isododecane (INCI), Undecane (INCI), Dodecane (INCI), Isohexadecane (INCI), hydrogenated polyisobutene (INCI: Hydrogenated Polyisobutene), Squalane (INCI), Mineral Oil (INCI), Coconut Alkanes (INCI), and (C13-15) Alkane (INCI).

Examples of the ester oils include diisobutyl adipate (INCI: Diisobutyl Adipate), dihexyldecyl adipate (labeling name), diheptylundecyl adipate (INCI: Diheptylundecyl Adipate), alkyl glycol monoisostearates, such as isostearyl isostearate (INCI: Isostearyl Isostearate), isocetyl isostearate (INCI: Isocetyl Isostearate), trimethylolpropane triisostearate (INCI: Trimethylolpropane Triisostearate), glycol diethylhexanoate (INCI: Glycol Diethylhexanoate), cetyl ethylhexanoate (INCI: Cetyl Ethylhexanoate), trimethylolpropane triethylhexanoate (INCI: Trimethylolpropane Triethylhexanoate), pentaerythrityl tetraethylhexanoate (INCI: Pentaerythrityl Tetraethylhexanoate), octyldodecyl esters, such as octyldodecyl stearoyloxystearate (INCI: Octyldodecyl Stearoyl Stearate), oleyl oleate (INCI: Oleyl Oleate), octyldodecyl oleate (INCI: Octyldodecyl Oleate), decyl oleate (INCI: Decyl Oleate), neopentyl glycol dioctanoate (INCI: Neopentyl Glycol Diethylhexanoate), neopentyl glycol dicaprate (INCI: Neopentyl Glycol Dicaprate), diisostearyl malate (INCI: Diisostearyl Malate), triethyl citrate (INCI: Triethyl Citrate), diethylhexyl succinate (INCI: Diethylhexyl Succinate), amyl acetate (INCI: Amyl Acetate), ethyl acetate (INCI: Ethyl Acetate), butyl acetate (INCI: Butyl Acetate), isocetyl stearate (INCI: Isocetyl Stearate), butyl stearate (INCI: Butyl Stearate), diisopropyl sebacate (INCI: Diisopropyl Sebacate), diethylhexyl sebacate (INCI: Diethylhexyl Sebacate), cetyl lactate (INCI: Cetyl Lactate), myristyl lactate (INCI: Myristyl Lactate), isononyl isononanoate (INCI: Isononyl Isononanoate), isotridecyl isononanoate (INCI: Isotridecyl Isononanoate), palmitates, such as isopropyl palmitate (INCI: Isopropyl Palmitate), ethylhexyl palmitate (INCI: Ethylhexyl Isopalmitate), and hexyldecyl palmitate (INCI: Isocetyl Palmitate, Hexyldecyl Palmitate), cholesteryl hydroxystearate (INCI: Cholesteryl Hydroxystearate), myristates, such as isopropyl myristate (INCI: Isopropyl Myristate), octyldodecyl myristate (INCI: Octyldodecyl Myristate), and myristyl myristate (INCI: Myristyl Myristate), ethylhexyl laurate (INCI: Ethylhexyl Laurate), hexyl laurate (INCI: Hexyl Laurate), dioctyldodecyl lauroyl glutamate (INCI: Dioctyldodecyl Lauroyl Glutamate), and isopropyl lauroyl sarcosinate (INCI: Isopropyl Lauroyl Sarcosinate).

Examples of the glyceride oils include Triethylhexanoin (INCI), glyceryl tri(caprylate/caprate) (INCI: Caprylic/Capric Triglyceride), Cocoglycerides (INCI), triglyceryl (caprylate/caprate/succinate) (INCI: Caprylic/Capric/Succinic Triglyceride), and (Caprylic/Capric) Glycerides (INCI).

Examples of the natural animal and plant oils and fats, and the semi-synthetic oils and fats include avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), insect wax, perilla oil (labeling name), perilla seed oil, olive oil (INCI: Olea Europaea (Olive) Fruit Oil), cacao butter, kapok wax, Torreya California oil (INCI: Torreya Californica (California Nutmeg) Oil), citronella grass oil (INCI: Cymbopogon Nardus (Citronella) Oil), kaya seed oil (INCI: Torreya Nucifera Seed Oil), carnauba wax (INCI: Copernicia Cerifera (Carnauba) Wax), liver oil, candelilla wax (INCI: Euphorbia Cerifera (Candelilla) Wax), refined candelilla wax, beef tallow, beef foot fat, beef bone fat, hydrogenated beef tallow, kyounin oil (INCI: Kyounin Yu), sperm wax, hydrogenated oil, wheat germ oil (INCI: Triticum Vulgare (Wheat) Germ Oil), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sugarcane wax (INCI: Saccharum Officinarum (Sugarcane) Wax), camellia oil (INCI: Camellia Kissi Seed Oil), safflower oil (INCI: Carthamus Tinctorius (Safflower) Seed Oil), shea butter (INCI: Butyrospermum Parkii (Shea Butter)), Aleurites Fordi oil, cinnamon oil, jojoba wax, Squalane (INCI), Squalene (INCI), shellac wax, turtle oil (INCI: Turtle Oil), soybean oil (INCI: Glycine Soja (Soybean) Oil), tea seed oil (INCI: Camellia Sinensis Seed Oil), camellia oil (INCI: Camellia Japonica Seed Oil), evening primrose oil (INCI: Oenothera Biennis (Evening Primrose) Oil), corn germ oil (INCI: Zea Mays (Corn) Germ Oil), lard (INCI: Lard), rapeseed oil, Japanese tung oil, rice bran wax (INCI: Oryza Sativa (Rice) Bran Wax), germ oil, horse tallow (INCI: Horse Fat), persic oil (labeling name), palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), hydrogenated castor oil, castor oil fatty acid methyl ester, sunflower oil (INCI: Helianthus Annuus (Sunflower) Seed Oil), grape seed oil (INCI: Vitis Vinifera (Grape) Seed Oil), bayberry wax, jojoba seed oil (INCI: Simmondsia Chinensis (Jojoba) Seed Oil), macadamia seed oil (INCI: Macadamia Ternifolia Seed Oil), macadamia nut oil (INCI: Macadamia Integrifolia Seed Oil) , beeswax (INCI: Beeswax), mink oil, meadowfoam oil (INCI: Limnanthes Alba (Meadowfoam) Seed Oil), cottonseed oil (INCI: Gossypium Herbaceum (Cotton) Seed Oil), cotton wax, Japan wax (INCI: Rhus Succedanea Fruit Wax), Japan wax kernel oil, montan wax (INCI: Montan Wax), coconut oil (INCI: Cocos Nucifera (Coconut) Oil), hydrogenated coconut oil, glyceryl tricocoate, mutton tallow, peanut oil (INCI: Arachis Hypogaea (Peanut) Oil), lanolin (INCI: Lanolin), liquid lanolin (INCI: Lanolin Oil), reduced lanolin, Lanolin Alcohol (INCI), hard lanolin, lanolin acetate, lanolin alcohol acetate (INCI: Acetylated Lanolin Alcohol), isopropyl lanolate (INCI: Isopropyl Lanolate), POE lanolin alcohol ether, POE lanolin alcohol acetate, polyethylene glycol lanolate, POE hydrogenated lanolin alcohol ether, and egg yolk oil (INCI: Egg Oil). POE indicates polyoxyethylene.

Examples of the higher alcohols include Lauryl Alcohol (INCI), Myristyl Alcohol (INCI), palmityl alcohol, Stearyl Alcohol (INCI), Behenyl Alcohol (INCI), hexadecyl alcohol, Oleyl Alcohol (INCI), Isostearyl Alcohol (INCI), hexyldodecanol, Octyldodecanol (INCI), cetostearyl alcohol, 2-decyltetradecynol, Cholesterol (INCI), Phytosterol (INCI), POE cholesterol ether, monostearyl glycerin ether (batyl alcohol), and monooleyl glyceryl ether (selachyl alcohol).

The amount of the oil (B) is variable depending on the formulation, but is 1 to 90% by mass, preferably 5 to 70% by mass, in the cosmetic. The amount may be 20 to 50% by mass.

### [Powders (C)]

The powder (C) may be any powder used in common cosmetics, regardless of its shape (spherical, acicular, plate-like, etc.), particle size (aerosol, fine particles, pigment-grade, etc.), and particle structure (porous, nonporous, etc.). A single kind, or two or more kinds of powders may be used. Examples of the powders include inorganic powders, organic powders, surfactant metal salt powders, colored pigments, pearl pigments, metal powder pigments, tar dyes, and natural dyes.

Examples of the inorganic powders include titanium dioxide (INCI: Titanium Dioxide), zirconium oxide (INCI: Zirconium Dioxide), zinc oxide (INCI: Zinc Oxide), cerium oxide (INCI: Cerium Oxide), magnesium oxide (Magnesium Oxide), barium sulfate (Barium Sulfate), calcium sulfate (Calcium Sulfate), magnesium sulfate (Magnesium Sulfate), calcium carbonate (Calcium Carbonate), magnesium carbonate (Magnesium Carbonate), talc (INCI: Talc), mica (INCI: Mica), kaolin (INCI: Kaolin), sericite (Sericite), titanium mica (Titanate Mica), silica (INCI: Silica), hydrous silica (INCI: Hydrated Silica), aluminum silicate (INCI: Aluminum Silicate), magnesium silicate (INCI: Magnesium Silicate), magnesium aluminum silicate (INCI: Magnesium Aluminum Silicate), calcium silicate (INCI: Calcium Silicate), hydroxyapatite (INCI: Hydroxyapatite), bentonite (INCI: Bentonite), montmorillonite (INCI: Montmorillonite), hectorite (INCI: Hectorite), zeolite (INCI: Zeolite), alumina (INCI: Alumina), aluminum hydroxide (Aluminum Hydroxide), and boron nitride (INCI: Boron Nitride). Among these, one or more selected from titanium dioxide, zinc oxide, and colored pigments are preferable.

Examples of the organic powders include polyamide (Polyamide), polyester (Polyester), polyethylene (INCI: Polyethylene), polypropylene (INCI: Polypropylene), polystyrene (INCI: Polystyrene), polyurethane (Polyurethane), polymethyl methacrylate (INCI: Polymethyl Methacrylate), cellulose (INCI: Cellulose), silk (Silk), nylon (Nylon), and silicones (INCI: Vinyl Dimethicone/Methicone Crosspolymer, Polymethylsilsesquioxane, Vinyl Dimethicone/Methicone Silsesquioxane Crosspolymer, Diphenyl Dimethicone/Vinyl Diphenyl Dimethicone/Silsesquioxane Crosspolymer, Polysilicone-1 Crosspolymer, Polysilicone-22).

Examples of the surfactant metal salt powders (metal soaps) include zinc stearate (INCI: Zinc Stearate), aluminum stearate (Aluminum Stearate), calcium stearate (Calcium Stearate), magnesium stearate (Magnesium Stearate), zinc myristate (INCI: Zinc Myristate), and magnesium myristate (Magnesium Myristate).

Examples of the colored pigments include inorganic red pigments, such as iron oxide (INCI: Iron Oxide), iron hydroxide, and iron titanate, inorganic brown pigments, such as γ-iron oxide, inorganic yellow pigments, such as yellow iron oxide (Yellow Oxide of Iron) and loess (INCI: Yellow Ocher), inorganic black pigments, such as black iron oxide (Black Oxide of Iron) and carbon black (INCI: Carbon Black), inorganic purple pigments, such as manganese violet (INCI: Manganese Violet), inorganic green pigments, such as chromium hydroxide (INCI: Chromium Hydroxide Green), chromium oxide (INCI: Chromium Oxide Green), and cobalt titanate (INCI: Cobalt Titanium dioxide), inorganic blue pigments, such as iron blue (Iron Blue) and ultramarine (Ultramarine), and synthetic resin powders that are combined with these powders.

Examples of the pearl pigments include titanium dioxide-coated mica, titanium dioxide-coated mica, bismuth oxychloride, titanium dioxide-coated bismuth oxychloride, titanium dioxide-coated talc, fish scale flakes, and titanium dioxide-coated colored mica.

Examples of the metal powder pigments include aluminum (Aluminum) powder and copper powder (INCI: Copper Powder).

Examples of the tar dyes include Red #3 (Erythrosine), Red #104 (Phloxine B), Red #106 (Acid red), Red #201 (Lithol rubine B), Red #202 (Lithol rubine BCA), Red #204 (Lake red CBA), Red #205 (Lithol red), Red #220 (Deep maroon), Red #226 (Helindone pink CN), Red #227 (Fast acid magenta), Red #228 (Permatone red), Red #230 (Eosine YS), Red #401 (Violamine R), Red #505 (Oil red XO), Yellow #4 (Tartrazin), Yellow #5 (Sunset yellow FCF), Yellow #202 (Uranine K), Yellow #203 (Quinoline yellow WS), Yellow #204 (Quinoline yellow SS), Yellow #401 (Hanza yellow), Blue #1 (Brilliant blue FCF), Blue #2 (Indigo carmine), Blue #201 (Indigo), Blue #404 (Phthalocyanine blue), Green #3 (Fast green FCF), Green #201 (Alizarine cyanine green F), Green #204 (Pyranine Conc), Green #205 (Light green SF yellowish), Orange #201 (Dibromofluorescein), Orange #203 (Permanent orange), Orange #204 (Benzidine orange G), Orange #206 (Diiodofluorescein), and Orange #207 (Erythrosine yellouwish NA).

Examples of the natural dyes include cochineal (Cochineal), laccaic acid (INCI: Laccaic Acid), carthamin (Carthamin), brazilin (Brazilin), and crocin (Crocin). Among those described above, one or more selected from titanium dioxide, zinc oxide, and colored pigments are preferable.

These powders may be in the form of composite powders, or may be treated with, for example, general oils, silicone oils, fluorine compounds, and surfactants. Where necessary, the powders may be treated with alkyl groups having a hydrolyzable silyl group or a hydrogen atom directly bonded to a silicon atom. Where necessary, use may be made of linear and/or branched organopolysiloxanes having a hydrolyzable silyl group or a hydrogen atom directly bonded to a silicon atom; linear and/or branched organopolysiloxanes having a hydrolyzable silyl group or a hydrogen atom directly bonded to a silicon atom, and co-modified with a long-chain alkyl; linear and/or branched organopolysiloxanes having a hydrolyzable silyl group or a hydrogen atom directly bonded to a silicon atom, and co-modified with a polyoxyalkylene; and acrylic-silicone copolymers having a hydrolyzable silyl group or a hydrogen atom directly bonded to a silicon atom. To obtain good handling, the powder is preferably dispersed into a liquid, a paste, or a solid.

The amount of the powder (C) is variable depending on the formulation, but is 2 to 95% by mass, preferably 5 to 70% by mass, in the cosmetic.

### [Organic UV absorbers (D)]

In addition to the components (A) to (C) described above, the cosmetic of the present invention may include an organic UV absorber (D) as an optional component. The organic UV absorber (D) may be any solid, semi-solid, or liquid organic UV absorber that is used in common cosmetics. Such organic UV absorbers may be used singly, or two or more may be used in combination.

Specific examples include Homosalate (INCI), Octocrylene (INCI), t-butyl methoxydibenzoylmethane (INCI: Butyl Methoxydibenzoylmethane), ethylhexyl salicylate (INCI: Ethylhexyl Salicylate), diethylamino hydroxybenzoyl hexyl benzoate (INCI: Diethylamino Hydroxybenzoyl Hexyl Benzoate), oxybenzone-6 (INCI: Benzophenone-6), oxybenzone-9 (INCI: Benzophenone-9), oxybenzone-1 (INCI: Benzophenone-1), Polysilicone-15 (INCI), octyl dimethoxybenzylidenedioxoimidazolidinepropionate (INCI: Ethylhexyl Dimethoxybenzylidene Dioxoimidazolidine Propionate), Oxybenzone-2 (INCI: Benzophenone-2), terephthalylidene dicamphor sulfonic acid (INCI: Terephthalylidene Dicamphor Sulfonic Acid), Ethylhexyl Triazone (INCI), methyl bis(trimethylsiloxy)silylisopentyl trimethoxycinnamate (INCI: Isopentyl Trimethoxycinnamate Trisiloxane), Drometrizole Trisiloxane (INCI), ethylhexyl dimethyl PABA (INCI: Ethylhexyl Dimethyl PABA), isopropyl p-methoxycinnamate (INCI: Isopropyl Methoxycinnamate), ethylhexyl methoxycinnamate (INCI: Ethylhexyl Methoxycinnamate), bis-ethylhexyloxyphenol methoxyphenyl triazine (INCI: Bis-Ethylhexyloxyphenol Methoxyphenyl Triazine), oxybenzone-3 (INCI: Benzophenone-3), oxybenzone-4 (INCI: Benzophenone-4), oxybenzone-5 (INCI: Benzophenone-5), phenylbenzimidazole sulfonic acid (INCI: Phenylbenzimidazole Sulfonic Acid), methylene bis-benzotriazolyl tetramethylbutylphenol (INCI: Methylene Bis-Benzotriazolyl Tetramethylbutylphenol), glyceryl ethylhexanoate dimethoxycinnamate (INCI: Glyceryl Ethylhexanoate Dimethoxycinnamate), glyceryl PABA (INCI: Glyceryl PABA), diisopropyl methyl cinnamate (INCI: Diisopropyl Methyl Cinnamate), Cinoxate (INCI), and ethylhexyl dimethoxybenzylidene dioxoimidazolidine propionate (INCI: Ethylhexyl Dimethoxybenzylidene Dioxoimidazolidine Propionate).

A UVA absorber (such as, for example, diethylamino hydroxybenzoyl hexyl benzoate (INCI: Diethylamino Hydroxybenzoyl Hexyl Benzoate)) and a UVB absorber (such as, for example, ethylhexyl methoxycinnamate (INCI: Ethylhexyl Methoxycinnamate)) may be used in combination, or absorbers belonging to the same type may be combined appropriately.

When the component (D) is added, the amount thereof is variable depending on the formulation, but is preferably 0.1 to 30% by mass, more preferably 1 to 15% by mass, in the cosmetic.

The cosmetic may include a surfactant (E) and water, and be an emulsion. The form of the emulsion is not particularly limited. When water is added, the amount thereof is variable depending on the formulation, but is preferably 1 to 95% by mass, more preferably 20 to 80% by mass, in the cosmetic.

### [Surfactants (E)]

The surfactants may be, for example, anionic, cationic, nonionic, or ampholytic. Any surfactants that are used in common cosmetics may be used without limitation.

Examples of the anionic surfactants include fatty acid soaps, such as sodium stearate and triethanolamine palmitate, alkyl ether carboxylic acids and salts thereof, amino acid-fatty acid condensate salts, alkane sulfonate salts, alkene sulfonate salts, sulfonate salts of fatty acid esters, sulfonate salts of fatty acid amides, formalin-condensed sulfonate salts, sulfate salts, such as alkyl sulfate salts, secondary higher alcohol sulfate salts, alkyl and allyl ether sulfate salts, sulfate salts of fatty acid esters, sulfate salts of fatty acid alkylolamides, and sulfated castor oil, alkyl phosphate salts, ether phosphate salts, alkyl allyl ether phosphate salts, amide phosphate salts, N-acyl lactate salts, N-acyl sarcosine salts, and N-acyl amino acid surfactants. Examples of the cationic surfactants include amine salts, such as alkyl amine salts, and polyamine and amino alcohol fatty acid derivatives, alkyl quaternary ammonium salts, aromatic quaternary ammonium salts, pyridinium salts, and imidazolium salts.

Examples of the nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, methylglucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesteryl ether, linear or branched polyoxyalkylene-modified organopolysiloxanes, linear or branched polyoxyalkylene·alkyl co-modified organopolysiloxanes, linear or branched polyglycerin-modified organopolysiloxanes, linear or branched polyglycerin·alkyl co-modified organopolysiloxanes, alkanolamides, sugar ethers, and sugar amides. Examples of the ampholytic surfactants include betaine, phosphatidylcholine, aminocarboxylate salts, imidazoline derivatives, and amidoamine compounds.

Of the above surfactants, preferred surfactants are linear or branched organopolysiloxanes having a polyoxyalkylene chain or a polyglycerin chain in the molecule, and such linear or branched organopolysiloxanes further having a C₆-C₂₀ long-chain alkyl group. In these surfactants, the content of hydrophilic polyoxyalkylene groups or polyglycerin residues is preferably 10 to 85% by mass of the molecule.

When the surfactant other than the components (A) is added, the amount thereof is preferably 0.1 to 20% by mass, more preferably 0.2 to 10% by mass, in the cosmetic.

The cosmetic of the present invention may contain appropriate amounts of optional components other than the components described above without impairing the advantageous effects of the present invention. Examples of such optional components include the following. The exemplary optional components of each category described below may be used singly, or two or more may be used in combination, and are selected appropriately in accordance with the target formulation and purpose.

### • Compounds having an alcoholic hydroxyl group in the molecular structure

Exemplary compounds having an alcoholic hydroxyl group include lower alcohols, such as ethanol (INCI: Alcohol) and isopropanol (INCI: Isopropyl Alcohol), sugar alcohols, such as Sorbitol (INCI) and Maltose (INCI), sterols, such as Cholesterol (INCI), sitosterol (INCI: Beta-Sitosterol), Phytosterol (INCI), and Lanosterol (INCI), and polyhydric alcohols, such as Butylene Glycol (INCI), Propylene Glycol (INCI), dibutylene glycol, and Pentylene Glycol (INCI). When the compound having an alcoholic hydroxyl group in the molecular structure is added, the amount thereof is preferably in the range of 0.1 to 98% by mass in the cosmetic.

### • Water-soluble or water-swellable polymer compounds

Exemplary water-soluble or water-swellable polymer compounds include plant-derived polymer compounds, such as gum arabic (INCI: Acacia Senegal Gum), tragacanth gum tree gum (INCI: Astragalus Gummifer Gum), galactan, carob gum, guar gum (INCI: Cyamopsis Tetragonoloba (Guar) Gum), karaya gum (INCI: Sterculia Urens Gum), carrageenan (INCI: Chondrus Crispus (Carrageenan)), Pectin (INCI), agar (INCI: Agar), quince seed (marmelo) (INCI: Pyrus Cydonia Seed), rice starch (INCI: Oryza Sativa (Rice) Starch), wheat starch (INCI: Triticum Vulgare (Wheat) Starch), potato starch (INCI: Solanum Tuberosum (Potato) Starch), corn starch, algae colloids, tragacanth gum (INCI: Astragalus Gummifer Gum), and locust bean gum; microorganism-derived polymer compounds, such as Xanthan Gum (INCI), Dextran (INCI), Succinoglycan (INCI), and Pullulan (INCI); animal-derived polymer compounds, such as Collagen (INCI), Casein (INCI), Albumin (INCI), and Gelatin (INCI); starch-based polymer compounds, such as sodium carboxymethyl starch (INCI: Sodium Carboxymethyl Starch) and hydroxypropyl starch (INCI: Hydroxypropyl Starch); cellulosic polymer compounds, such as Methylcellulose (INCI), Ethylcellulose (INCI), Methylhydroxypropylcellulose (INCI), carboxymethylcellulose, hydroxymethylcellulose, Hydroxypropylcellulose (INCI), Nitrocellulose (INCI), sodium cellulose sulfate (INCI: Sodium Cellulose Sulfate), sodium carboxymethylcellulose, crystalline cellulose (INCI: Microcrystalline Cellulose), and cellulose powder; alginic acid-based polymer compounds, such as sodium alginate (INCI: Algin) and propylene glycol alginate (INCI: Propylene Glycol Alginate); vinyl-based polymer compounds, such as polyvinyl methyl ether and carboxyvinyl polymer (INCI: Carbomer); polyoxyethylene-based polymer compounds; polyoxyethylene-polyoxypropylene copolymer-based polymer compounds; acrylic polymers, such as sodium polyacrylate (INCI: Sodium Polyacrylate), polyethylacrylate (INCI: Polyethylacrylate), Polyacrylamide (INCI), and acryloyldimethyltaurate copolymer; other synthetic water-soluble polymer compounds, such as polyethyleneimine and cationic polymers; and inorganic water-soluble polymers, such as Bentonite (INCI), magnesium aluminum silicate (INCI: Magnesium Aluminum Silicate), Montmorillonite (INCI), beidellite, nontronite, saponite, Hectorite (INCI), and silicic anhydride. The water-soluble polymer compounds also include film-forming agents, such as Polyvinyl Alcohol (INCI) and PVP (INCI). When the water-soluble or water-swellable polymer compound is added, the amount thereof is preferably in the range of 0.1 to 25% by mass in the cosmetic.

### • Silicone resins

Preferred silicone resins are acrylic silicone resins in the form of acrylic/silicone graft or block copolymers. Use may be made of acrylic silicone resins that contain in the molecule one or more selected from pyrrolidone moieties, long-chain alkyl moieties, polyoxyalkylene moieties, fluoroalkyl moieties, and anionic moieties, such as carboxylic acids.

Furthermore, the silicone resins are preferably silicone network compounds that include resins composed of R₃SiO_{1/2} units and SiO_{4/2} units, resins composed of R₃SiO_{1/2} units, R₂SiO_{2/2} units, and SiO_{4/2} units, resins composed of R₃SiO_{1/2} units and RSiO_{3/2} units, resins composed of R₃SiO_{1/2} units, R₂SiO_{2/2} units, and RSiO_{3/2} units, or resins composed of R₃SiO_{1/2} units, R₂SiO_{2/2} units, RSiO_{3/2} units, and SiO_{4/2} units (wherein R denotes a monovalent organic group). Use may be made of silicone network compounds that contain in the molecule one or more selected from pyrrolidone moieties, long-chain alkyl moieties, polyoxyalkylene moieties, fluoroalkyl moieties, and amino moieties.

The silicone resins further include silicone-modified pullulans and silicone-modified norbornenes. When the silicone resin is added, the amount thereof is preferably 0.1 to 20% by mass, more preferably 1 to 10% by mass, in the cosmetic.

### • Compositions consisting of a crosslinked organopolysiloxane and an oil that is liquid at room temperature

The crosslinked organopolysiloxane is preferably swollen by containing its own weight or more of the liquid oil. For example, the liquid oil may be any of the silicone oils, the hydrocarbon oils, the ester oils, the natural animal and plant oils, the semi-synthetic oils, and the fluorochemical oils that are described hereinabove and are liquid. Examples include low-viscosity silicone oils, liquid paraffins, hydrocarbon oils, such as squalane, isododecane, and isohexadecane, glyceride oils, such as trioctanoin, ester oils, such as isotridecyl isononanoate, N-acylglutamic acid esters, and lauroyl sarcosine esters, and natural animal and plant oils, such as macadamia nut oil, each having a kinematic viscosity of 0.65 to 100.0 mm²/s as measured at 25°C with a Cannon-Fenske viscometer in accordance with JIS Z 8803: 2011. It is preferable that the crosslinking agent in this crosslinked organopolysiloxane have two or more vinyl reactive sites in the molecule and form a crosslinked structure by reacting with hydrogen atoms directly bonded to silicon atoms. Examples of the agents having two or more vinyl reactive sites in the molecule include organopolysiloxanes having two or more vinyl groups in the molecule, polyoxyalkylenes having two or more allyl groups in the molecule, polyglycerols having two or more allyl groups in the molecule, and α,ω-alkenyldienes. Use may be made of crosslinked organopolysiloxanes that contain in the crosslinked molecule at least one selected from the group consisting of polyoxyalkylene moieties, polyglycerol moieties, long-chain alkyl moieties, alkenyl moieties, aryl moieties, and fluoroalkyl moieties. When the composition consisting of a crosslinked organopolysiloxane and an oil that is liquid at room temperature is added, the amount thereof is preferably 0.1 to 80% by mass, more preferably 1 to 50% by mass, in the cosmetic.

### • Silicone waxes

Silicone waxes are silicone-modified olefin waxes that are obtained by the addition reaction of an olefin wax obtained from an α-olefin and a diene, and having an unsaturated group, with an organohydrogenpolysiloxane having one or more hydrosilyl groups in the molecule. The α-olefin in the olefin wax is preferably a C₂-C₁₂ α-olefin, such as ethylene, propylene, 1-butene, 1-hexene, or 4-methyl-1-pentene, and the diene is preferably butadiene, isoprene, 1,4-hexadiene, vinylnorbornene, ethylidenenorbornene, or dicyclopentadiene. The organohydrogenpolysiloxane having a hydrosilyl group may have, for example, a linear structure or a siloxane-branched structure.

Furthermore, the cosmetic of the present invention may contain components used in common cosmetics, with examples including oil-soluble gelling agents, antiperspirants, moisturizers, antibacterial and preservative agents, antibacterial agents, fragrances, salts, antioxidants, acidity regulators, chelating agents, refreshing agents, anti-inflammatory agents, skin modifying ingredients (such as brightening or whitening agents, cell activators, anti-skin-roughening agents, blood flow enhancing agents, skin astringents, and antiseborrheic agents), vitamins, amino acids, nucleic acids, hormones, and hair-setting ingredients.

For example, the oil-soluble gelling agents may be selected from metal soaps, such as aluminum stearate, magnesium stearate (INCI: Magnesium Stearate), and zinc myristate (INCI: Zinc Myristate), amino acid derivatives, such as N-lauroyl-L-glutamic acid (INCI: Lauroyl Glutamic Acid) and α,γ-di-n-butylamine, dextrin fatty acid esters, such as dextrin palmitate (INCI: Dextrin Palmitate), dextrin stearate (INCI: Dextrin Stearate), and dextrin 2-ethylhexanoate palmitate (INCI: Dextrin Palmitate/Ethylhexanoate), sucrose fatty acid esters, such as sucrose palmitate (INCI: Sucrose Palmitate) and sucrose stearate (INCI: Sucrose Stearate), fructooligosaccharide fatty acid esters, such as fructooligosaccharide stearate and fructooligosaccharide 2-ethylhexanoate, benzylidene derivatives of sorbitol, such as monobenzylidene sorbitol and dibenzylidene sorbitol, and organically modified clay minerals, such as dimethylbenzyldodecylammonium montmorillonite clay and dimethyldioctadecylammonium montmorillonite clay. The cosmetic of the present invention can achieve sufficient effects even without containing these oil-soluble gelling agents.

Examples of the antiperspirants include aluminum chlorohydrate (INCI: Aluminum Chlorohydrate), aluminum chloride (INCI: Aluminum Chloride), aluminum sesquichlorohydrate, zirconyl hydroxychloride, aluminum zirconium hydroxychloride, and aluminum zirconium glycine complex.

Examples of the moisturizers include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate salt, polyoxyethylene methyl glucoside, polyoxypropylene methyl glucoside, egg yolk lecithin, soybean lecithin, phosphatidylcholine, phosphatidylethanolamine, phosphatidylserine, phosphatidylglycerol, phosphatidylinositol, and sphingophospholipids.

Examples of the antibacterial and preservative agents include alkyl p-hydroxybenzoates, benzoic acid (INCI: Benzoic Acid), sodium benzoate (INCI: Sodium Benzoate), sorbic acid (INCI: Sorbic Acid), potassium sorbate (INCI: Potassium Sorbate), and Phenoxyethanol (INCI). Examples of the antibacterial agents include benzoic acid, salicylic acid, Phenol (INCI), sorbic acid, alkyl p-hydroxybenzoates, p-Chlorometacresol (INCI), Hexachlorophene (INCI), Benzalkonium Chloride (INCI), chlorhexidine dihydrochloride (INCI: Chlorhexidine Dihydrochloride), trichlorocarbanilide, photosensitizers, and phenoxyethanol.

Examples of the fragrances include natural fragrances and synthetic fragrances. Examples of the natural fragrances include plant fragrances separated from flowers, leaves, stems, pericarps, and the like; and animal fragrances, such as musk and civet. Examples of the synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

Examples of the salts include inorganic salts, organic acid salts, amine salts, and amino acid salts. Examples of the inorganic salts include sodium salts, potassium salts, magnesium salts, calcium salts, aluminum salts, zirconium salts, and zinc salts of such inorganic acids as hydrochloric acid, sulfuric acid, carbonic acid, and nitric acid. Examples of the organic acid salts include salts of such organic acids as acetic acid (INCI: Acetic Acid), dehydroacetic acid (INCI: Dehydroacetic Acid), citric acid (INCI: Citric Acid), malic acid (INCI: Malic Acid), succinic acid (INCI: Succinic Acid), ascorbic acid (INCI: Ascorbic Acid), and stearic acid (INCI: Stearic Acid). Examples of the amine salts and the amino acid salts include salts of such amines as triethanolamine (INCI: Triethanolamine), and salts of such amino acids as glutamic acid. Also useful are, for example, salts of hyaluronic acid (INCI: Hyaluronic Acid), chondroitin sulfate, aluminum zirconium glycine complex, and acid-alkali neutral salts used in cosmetic formulations.

Examples of the antioxidants include Tocopherol (INCI), p-t-butylphenol, Butylhydroxyanisole (INCI), Dibutylhydroxytoluene (INCI), and phytic acid.

Examples of the acidity regulators include lactic acid (INCI: Lactic Acid), citric acid (INCI: Citric Acid), glycolic acid (INCI: Glycolic Acid), succinic acid (INCI: Succinic Acid), tartaric acid (INCI: Tartaric Acid), dl-malic acid (INCI: Malic Acid), potassium carbonate (INCI: Potassium Carbonate), sodium hydrogencarbonate (INCI: Sodium Bicarbonate), and ammonium hydrogencarbonate (INCI: Ammonium Bicarbonate).

Examples of the chelating agents include Alanine (INCI), sodium edetate, sodium polyphosphate (INCI: Sodium Polyphosphate), sodium metaphosphate (INCI: Sodium Metaphosphate), and phosphoric acid.

Examples of the refreshing agents include L-menthol and camphor.

Examples of the anti-inflammatory agents include Allantoin (INCI), glycyrrhizic acid (INCI: Glycyrrhizic Acid) and salts thereof, glycyrrhetinic acid (INCI: Glycyrrhetinic Acid), stearyl glycyrrhetinate (INCI: Stearyl Glycyrrhetinate), tranexamic acid, and Azulene (INCI).

Examples of the skin modifying ingredients include brightening or whitening agents, such as placenta extract, Arbutin (INCI), Glutathione (INCI), and saxifraga extract (INCI: Saxifraga Sarmentosa Extract); cell activators, such as Royal Jelly (INCI), photosensitizers, cholesterol derivatives, and calf blood extract; anti-skin-roughening agents; blood flow enhancing agents, such as vanillylamide nonanoate, benzyl nicotinate (INCI: Benzyl Nicotinate), β-butoxyethyl nicotinate, Capsaicin (INCI), zingerone, cantharides tincture, Ichthammol (INCI), Caffeine (INCI), tannic acid (INCI: Tannic Acid), α-Borneol (INCI), tocopherol nicotinate (INCI: Tocopheryl Nicotinate), inositol hexanicotinate, cyclandelate, cinnarizine, tolazoline, acetylcholine, verapamil, cepharanthine, and γ-Oryzanol (INCI); skin astringents, such as zinc oxide and tannic acid; and antiseborrheic agents, such as sulfur and thianthol.

Examples of the vitamins include vitamin A family, such as vitamin A oil, Retinol (INCI), retinol acetate (INCI: Retinyl Acetate), and retinol palmitate (INCI: Retinyl Palmitate); vitamin B family, including vitamin B2 family, such as Riboflavin (INCI), riboflavin butyrate (INCI: Riboflavin Tetrabutyrate), and flavin adenine nucleotide (INCI: Disodium Flavine Adenine Dinucleotide), vitamin B6 family, such as pyridoxine hydrochloride, pyridoxine dioctanoate, and pyridoxine tripalmitate (INCI: Pyridoxine Tripalmitate), vitamin B12 and derivatives thereof, and vitamin B15 and derivatives thereof; vitamin C family, such as L-ascorbic acid (INCI: Ascorbic Acid), L-ascorbic acid dipalmitic acid esters, L-ascorbic acid-2-sodium sulfate (INCI: Disodium Ascorbyl Sulfate), and dipotassium L-ascorbic acid phosphoric acid diester; vitamin D family, such as Ergocalciferol (INCI) and Cholecalciferol (INCI); vitamin E family, such as α-Tocopherol (INCI), β-Tocopherol (INCI), γ-Tocopherol (INCI), dl-α-tocopherol acetate (INCI: Tocopheryl Acetate), dl-α-tocopherol nicotinate (INCI: Tocopheryl Nicotinate), and dl-α-tocopherol succinate (INCI: Tocopheryl Succinate); vitamin H; vitamin P; nicotinic acid derivatives, such as nicotinic acid, benzyl nicotinate, and nicotinamide; pantothenic acids, such as calcium pantothenate, D-pantothenyl alcohol, pantothenyl ethyl ether, and acetyl pantothenyl ethyl ether; and biotin.

Examples of the amino acids include Glycine (INCI), Valine (INCI), Leucine (INCI), Isoleucine (INCI), Serine (INCI), Threonine (INCI), Phenylalanine (INCI), Arginine (INCI), Lysine (INCI), aspartic acid (INCI: Aspartic Acid), glutamic acid (INCI: Glutamic Acid), Cystine (INCI), Cysteine (INCI), Methionine (INCI), and Tryptophan (INCI). Examples of the nucleic acids include deoxyribonucleic acid. Examples of the hormones include Estradiol (INCI) and Ethenyl Estradiol (INCI).

Examples of the hair-setting polymer compounds include ampholytic, anionic, cationic, and nonionic polymer compounds. Specific examples include polyvinylpyrrolidone polymer compounds, such as Polyvinylpyrrolidone (INCI) and Vinylpyrrolidone/Vinyl Acetate Copolymer (INCI); acidic vinyl ether polymer compounds, such as methyl vinyl ether/maleic anhydride alkyl half ester copolymers; acidic polyvinyl acetate polymers, such as vinyl acetate/crotonic acid copolymer; acidic acrylic polymer compounds, such as (meth)acrylic acid/alkyl (meth)acrylate copolymers and (meth)acrylic acid/alkyl (meth)acrylate/alkyl acrylamide copolymers; and ampholytic acrylic polymer compounds, such as N-methacryloylethyl-N,N-dimethylammonium·α-N-methylcarboxybetaine/alkyl (meth)acrylate copolymers and hydroxypropyl (meth)acrylate/butylaminoethyl methacrylate/octyl acrylamide copolymer. Furthermore, naturally occurring polymer compounds, such as cellulose and derivatives thereof, and keratin, collagen, and derivatives thereof, may also be suitably used.

### [Cosmetics]

By including the carboxylic acid-modified silicone offering excellent powder dispersibility and dispersion stability, the cosmetic can give a finish with uniform coloring, spreads lightly without resistance caused by aggregates at the time of application, fits well to the skin, and exhibits excellent stability over time. The type of the cosmetic is not particularly limited. Exemplary types include powders, oils, water-in-oil emulsions, oil-in-water emulsions, non-aqueous emulsions, and multiphase emulsions, such as W/O/W and O/W/O. The pH and the viscosity are selected appropriately in accordance with the formulation.

The viscosity of the cosmetic is not particularly limited in any formulation, but is preferably 1,000 to 300,000 mPa·s, and more preferably 1,000 to 200,000 mPa·s. In the present invention, the viscosity is a value measured at 25°C with a Brookfield viscometer. An example of the Brookfield viscometers is TVB-10, manufactured by Toki Sangyo Co., Ltd.

The cosmetic of the present invention may be produced by any method without limitation. A conventional method may be selected depending on the formulation. In the present invention, the components (A) to (C) and any optional components may be mixed at the time of producing the cosmetic. The components (A) to (C) may be formed into a dispersion before being added to the cosmetic. In this manner, the dispersibility and the dispersion stability of the powder can be further improved. The dispersion may be prepared by any known method without limitation. For example, use can be made of an appropriate stirring machine, crusher, mixer, or disperser, such as a Henschel mixer, a ball mill, a kneader, a planetary mixer, a ribbon blender, a dispersing mixer, a homomixer, a roll mill, or a bead mill. The dispersion exhibits a low viscosity when dispersibility is good. Thus, the dispersibility of the component (C) can be evaluated by measuring the viscosity. The viscosity of the dispersion is preferably less than 4,000 mPa·s, and more preferably 100 to 3,000 mPa·s. The total amount of the components (A) to (C) in the dispersion is preferably 50% by mass or more, more preferably 80% by mass or more, and even more preferably 90% by mass or more.

The form of the cosmetic may be selected from a variety of forms including liquid, milky lotion, cream, solid, paste, gel, powder, pressed, multi-layered, mousse, spray, stick, and pencil.

Examples of the cosmetics include skin care cosmetics, such as liquids, massage products, beauty serums, beauty oils, cleansers, deodorants, hand creams, lip balms, and wrinkle concealers; makeup cosmetics, such as makeup bases, concealers, face powders, powder foundations, liquid foundations, cream foundations, oil-based foundations, blushers, eye shadows, mascaras, eyeliners, eyebrows, and lip cosmetics; hair cosmetics, such as shampoos, conditioners, treatments, and setting agents; antiperspirants; and UV protection cosmetics, such as sunscreen oils, sunscreen milky lotions, and sunscreen creams. In particular, those cosmetics that contain water and are in the form of emulsions are suitable as makeup bases, liquid foundations, sunscreen milky lotions, sunscreen creams, and the like.

### EXAMPLES

The present invention will be described in detail below by presenting Synthesis Examples, Examples, and Comparative Examples. However, the present invention is not limited to the following Examples. In the following examples, "%" in the formulations indicates % by mass unless otherwise specified. The amounts of substances described with product names are the amounts of the products added. Carboxylic acid-modified silicone compounds are illustrated in Synthesis Examples 1 to 5, and cosmetics in Examples and Comparative Examples. In the tables, Synthesis Example 1 indicates the carboxylic acid-modified silicone obtained in Synthesis Example 1, and the same applies to other synthesis examples. In the tables, Comparative Structure 1 indicates the silicone having Comparative Structure 1, and the same applies to other comparative structures. The orders in which siloxane units are bonded are not limited to those illustrated below.

### Synthesis Examples: Carboxylic acid-modified silicones (A)

### [Synthesis Example 1]

A reactor was charged with 87 g of methylhydrogenpolysiloxane represented by formula (8), 24 g of allyl glycol, 20 g of isopropyl alcohol, and 0.1 g of a 0.5% toluene solution of chloroplatinic acid-vinyl siloxane complex. The mixture was reacted at 80°C for 3 hours. Subsequently, the mixture was distilled under reduced pressure at 110°C/400 Pa to give hydroxyl-containing dimethylpolysiloxane represented by formula (9). Next, 30 g of succinic anhydride, 50 g of tetrahydrofuran (hereinafter, THF), and 10 g of triethylamine were added to the product of formula (9), and the mixture was reacted at 70°C for 5 hours. After the completion of the reaction, 80 g of water was added and the system was washed with water. Water-washing was performed three more times, and the mixture was distilled under reduced pressure at 100°C/5 mm Hg to give carboxylic acid-modified silicone represented by formula (10). The product was a pale yellow liquid, and had a molecular weight of 1,300.

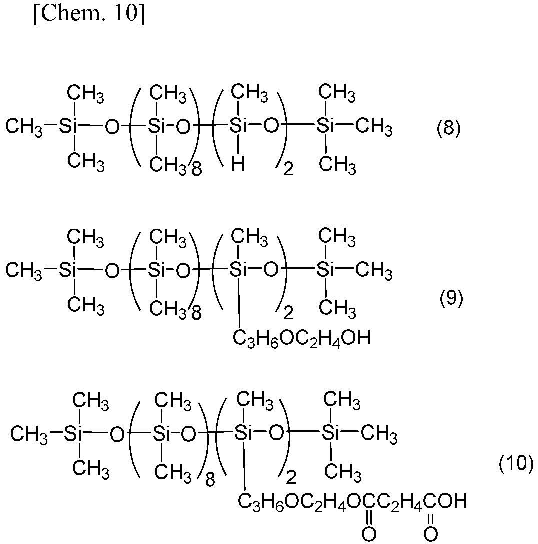

### [Synthesis Example 2]

A reactor was charged with 42 g of methylhydrogenpolysiloxane represented by formula (11), 77 g of the organopolysiloxane represented by formula (12), and 0.1 g of a 0.5% toluene solution of chloroplatinic acid-vinyl siloxane complex. The mixture was reacted at 80°C for 3 hours. Subsequently, 30 g of allyl glycol, 20 g of isopropyl alcohol, and 0.1 g of a 0.5% toluene solution of chloroplatinic acid-vinyl siloxane complex were added. The mixture was reacted at 80°C for 3 hours. Subsequently, the mixture was distilled under reduced pressure at 110°C/3 mm Hg to give hydroxyl-containing dimethylpolysiloxane represented by formula (13). Next, 30 g of succinic anhydride, 50 g of THF, and 10 g of triethylamine were added to the product of formula (13), and the mixture was reacted at 70°C for 5 hours. After the completion of the reaction, 80 g of water was added and the system was washed with water. Water-washing was performed three more times, and the mixture was distilled under reduced pressure at 100°C/5 mm Hg to give carboxylic acid-modified silicone of formula (14). The product was a pale yellow liquid, and had a molecular weight of 1,600.

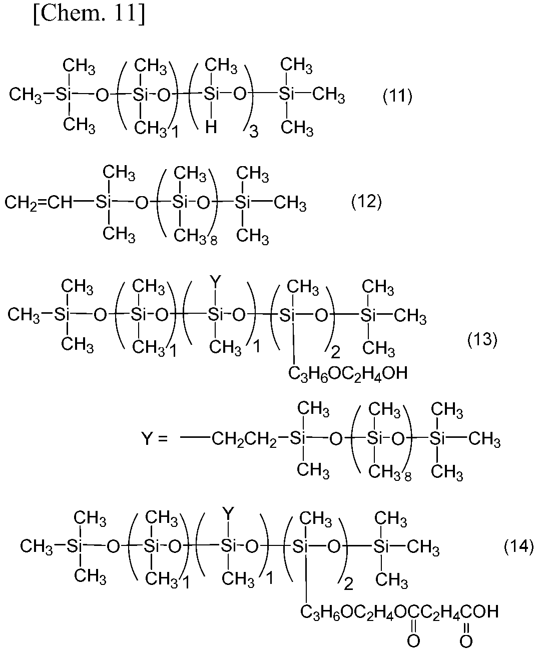

### [Synthesis Example 3]

A reactor was charged with 48 g of methylhydrogenpolysiloxane represented by formula (15), 50 g of 1-dodecene, and 0.1 g of a 0.5% toluene solution of chloroplatinic acid-vinyl siloxane complex. The mixture was reacted at 80°C for 3 hours. Subsequently, 15 g of allyl glycol, 10 g of isopropyl alcohol, and 0.1 g of a 0.5% toluene solution of chloroplatinic acid-vinyl siloxane complex were added, and the mixture was reacted at 80°C for 3 hours. Subsequently, the mixture was distilled under reduced pressure at 110°C/3 mm Hg to give hydroxyl-containing dimethylpolysiloxane of formula (16). Next, 15 g of succinic anhydride, 50 g of THF, and 10 g of triethylamine were added to the product of formula (16), and the mixture was reacted at 70°C for 5 hours. After the completion of the reaction, 80 g of water was added and the system was washed with water. Water-washing was performed three more times, and the mixture was distilled under reduced pressure at 100°C/5 mm Hg to give carboxylic acid-modified silicone of formula (17). The product was a pale yellow liquid, and had a molecular weight of 1,200.

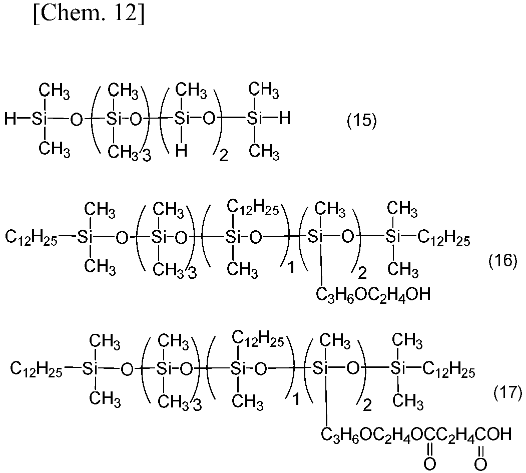

### [Synthesis Example 4]

A reactor was charged with 83 g of methylhydrogenpolysiloxane represented by formula (18), 62 g of allyl isostearyl ether, and 0.1 g of a 0.5% toluene solution of chloroplatinic acid-vinyl siloxane complex. The mixture was reacted at 80°C for 3 hours. Furthermore, 77 g of the organopolysiloxane represented by formula (12) and 0.1 g of a 0.5% toluene solution of chloroplatinic acid-vinyl siloxane complex were added, and the mixture was reacted at 80°C for 3 hours. Subsequently, 30 g of allyl glycol, 20 g of isopropyl alcohol, and 0.1 g of a 0.5% toluene solution of chloroplatinic acid-vinyl siloxane complex were added. The mixture was reacted at 80°C for 3 hours. Subsequently, the mixture was distilled under reduced pressure at 110°C/3 mm Hg to give hydroxyl-containing dimethylpolysiloxane represented by formula (19). Next, 30 g of succinic anhydride, 50 g of THF, and 10 g of triethylamine were added to the product of formula (19), and the mixture was reacted at 70°C for 5 hours. After the completion of the reaction, 200 g of water was added and the system was washed with water. Water-washing was performed three more times, and the mixture was distilled under reduced pressure at 100°C/5 mm Hg to give carboxylic acid-modified silicone of formula (20). The product was a pale yellow liquid, and had a molecular weight of 2,800.

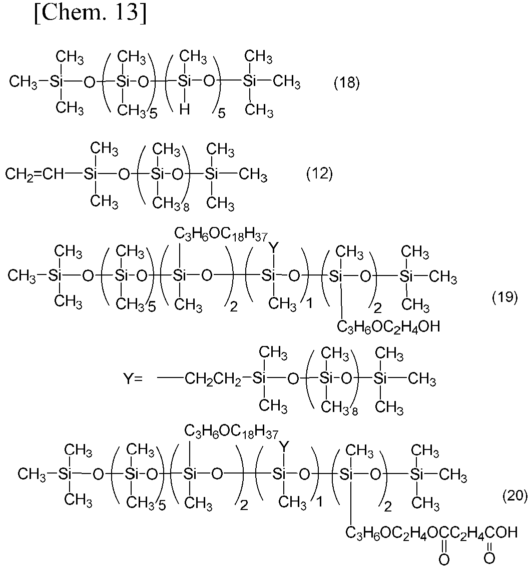

### [Synthesis Example 5]

A reactor was charged with 135 g of methylhydrogenpolysiloxane represented by formula (21), 45 g of 1-hexadecene, and 0.15 g of a 0.5% toluene solution of chloroplatinic acid-vinyl siloxane complex. The mixture was reacted at 80°C for 3 hours. Furthermore, 77 g of the organopolysiloxane represented by formula (12) and 0.1 g of a 0.5% toluene solution of chloroplatinic acid-vinyl siloxane complex were added, and the mixture was reacted at 80°C for 3 hours. Subsequently, 49 g of the compound of formula (22), 40 g of isopropyl alcohol, and 0.1 g of a 0.5% toluene solution of chloroplatinic acid-vinyl siloxane complex were added. The mixture was reacted at 80°C for 3 hours. Subsequently, the mixture was distilled under reduced pressure at 110°C/3 mm Hg to give hydroxyl-containing dimethylpolysiloxane represented by formula (23). Next, 30 g of succinic anhydride, 100 g of THF, and 10 g of triethylamine were added to the product of formula (23), and the mixture was reacted at 70°C for 5 hours. After the completion of the reaction, 300 g of water was added and the system was washed with water. Water-washing was performed three more times, and the mixture was distilled under reduced pressure at 100°C/5 mm Hg to give carboxylic acid-modified silicone of formula (24). The product was a pale yellow liquid, and had a molecular weight of 3,500.

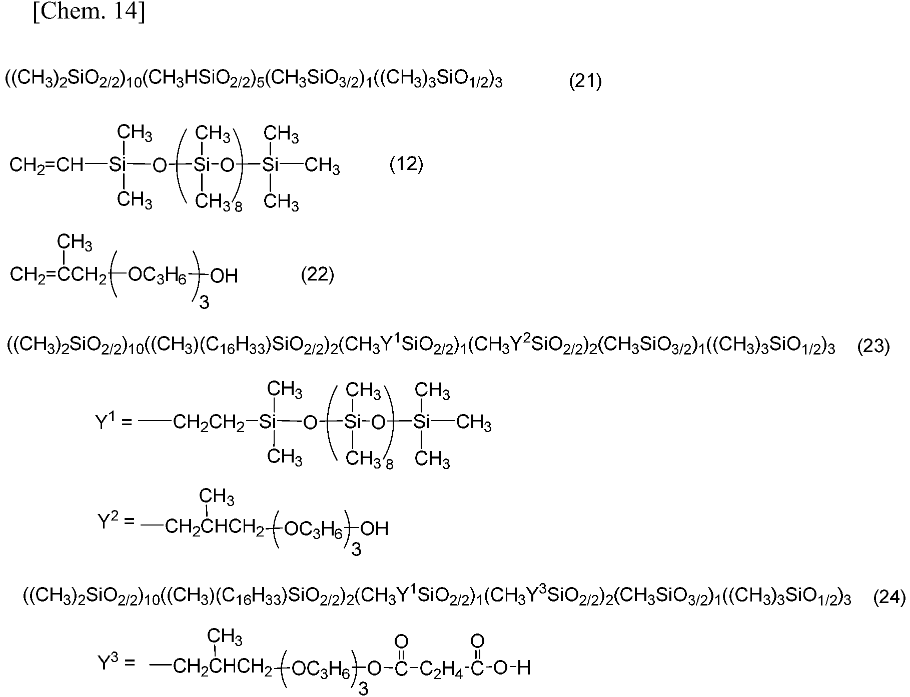

The molecular weight measured above is a value of weight average molecular weight determined by gel permeation chromatography (GPC) analysis using polystyrene standards under the following conditions.

### [Measurement conditions]

| | |
|---|---|
| Developing solvent: | Tetrahydrofuran (THF) |
| Flow rate: | 0.6 mL/min |
| Detector: | Differential refractive index detector (RI) |
| Columns: | TSK Guardcolumn Super H-H |
| | TSKgel SuperHM-N (6.0 mm I.D. × 15 cm × 1) |
| | TSKgel SuperH2500 (6.0 mm I.D. × 15 cm × 1) |
| | (all manufactured by Tosoh Corporation) |
| Column temperature: | 40°C |
| Sample injection volume: | 50 µL (0.3 mass% THF solution) |

### [Examples 1 to 15 and Comparative Examples 1 to 6]

Using a bead mill, the carboxylic acid-modified silicones obtained in Synthesis Examples 1 to 5 were formed into dispersions (slurries) in accordance with the formulations described in Table 1 and 2 below. The dispersions obtained were evaluated for dispersibility in the manner described below. Comparative Structure 1 used in Comparative Examples was the carboxylic acid-modified silicone of formula (25) below. Hereinafter, Synthesis Examples in the tables indicate the carboxylic acid-modified silicones obtained in the respective Synthesis Examples.

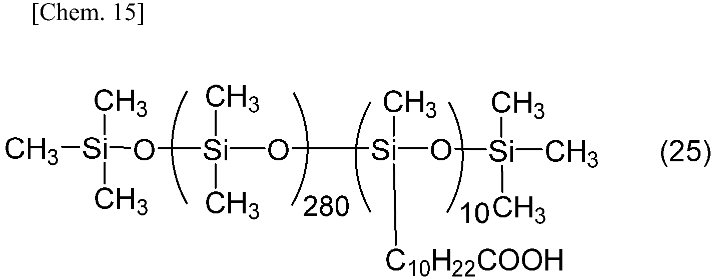

Comparative Structure 2 was the carboxylic acid-modified silicone of formula (26) below.

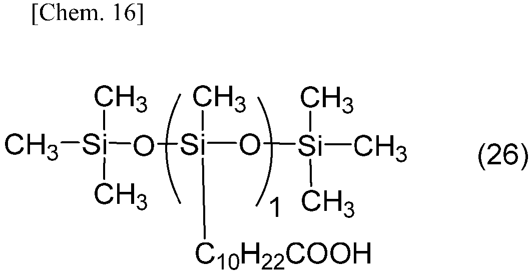

### [Evaluation of dispersion viscosity (dispersibility)]

In a glass bottle, 100 g of the dispersion obtained was stored at 25°C for 2 months. The viscosity of the dispersion immediately after preparation and after 2 months was measured at 25°C using a Brookfield viscometer (TVB-10, manufactured by Toki Sangyo Co., Ltd.) by the method described in JIS K7111-1: 1999. The results are shown according to the evaluation criteria below.

### [Evaluation criteria]

| | |
|---|---|
| ⊚: | 1,000 mPa·s or more and less than 2,000 mPa·s |
| ○: | 2,000 mPa·s or more and less than 4,000 mPa·s |
| Δ: | 4,000 mPa·s or more and less than 10,000 mPa·s |
| ×: | 10,000 mPa·s or more, no fluidity |

**[Table 1]**

| Formulation (%) | | Example | | | | | | | | | |
|---|---|---|---|---|---|---|---|---|---|---|---|
| | | 1 | 2 | 3 | 4 | 5 | 6 | 7 | 8 | 9 | 10 |
| (A) | Synthesis Example 1 | 10 | - | - | - | - | 5 | - | - | - | - |
| | Synthesis Example 2 | - | 10 | - | - | - | - | 5 | - | - | - |
| | Synthesis Example 3 | - | - | 10 | - | - | - | - | 5 | - | - |
| | Synthesis Example 4 | - | - | - | 10 | - | - | - | - | 5 | - |
| | Synthesis Example 5 | - | - | - | - | 10 | - | - | - | - | 5 |
| (B) Oil | Cyclopentasiloxane | - | - | 40 | 40 | 40 | - | - | 30 | 30 | 30 |
| | Dimethylpolysiloxane (2 mm²/s) | 40 | 40 | - | - | - | 30 | 30 | - | - | - |
| (C) Powder | STR-100A-LP *¹ | 50 | 50 | 50 | 50 | 50 | - | - | - | - | - |
| | FINEX-30-LP2 *² | - | - | - | - | - | 65 | 65 | 65 | 65 | 65 |
| Total | | 100 | | | | | | | | | |
| Dispersion viscosity | Immediately after preparation | ⊚ | ⊚ | ⊚ | ⊚ | ⊚ | ⊚ | ⊚ | ⊚ | ⊚ | ⊚ |
| | 2 months later | ⊚ | ⊚ | ○ | ○ | ○ | ⊚ | ⊚ | ⊚ | ○ | ○ |

**[Table 2]**

| Formulation (%) | | Example | | | | | Comparative Example | | | | | |
|---|---|---|---|---|---|---|---|---|---|---|---|---|
| | | 11 | 12 | 13 | 14 | 15 | 1 | 2 | 3 | 4 | 5 | 6 |
| (A) | Synthesis Example 1 | 3 | - | - | - | - | - | - | - | - | - | - |
| | Synthesis Example 2 | - | 3 | - | - | - | - | - | - | - | - | - |
| | Synthesis Example 3 | - | - | 3 | - | - | - | - | - | - | - | - |
| | Synthesis Example 4 | - | - | - | 3 | - | - | - | - | - | - | - |
| | Synthesis Example 5 | - | - | - | - | 3 | - | - | - | - | - | - |
| | Comparative structure 1 | - | - | - | - | - | 10 | - | 5 | - | 3 | - |
| | Comparative structure 2 | - | - | - | - | - | - | 10 | - | 5 | - | 3 |
| (B) Oil | Cyclopentasiloxane | - | - | - | - | - | 40 | 40 | 30 | 30 | - | - |
| | Isotridecyl isononanoate | 27 | 27 | 27 | 27 | 27 | - | - | - | - | 27 | 27 |
| (C) Powder | STR-100A-LP *1 | - | - | - | - | - | 50 | 50 | - | - | - | - |
| | FINEX-30-LP2 *2 | 70 | 70 | 70 | 70 | 70 | - | - | 65 | 65 | 70 | 70 |
| Total | | 100 | | | | | | | | | | |
| Dispersion viscosity | Immediately after preparation | ○ | ○ | ⊚ | ⊚ | ⊚ | × | Δ | × | Δ | Δ | Δ |
| | 2 months later | ○ | ○ | ⊚ | ⊚ | ⊚ | × | Δ | × | Δ | × | Δ |

Regarding *1 and *2 in Tables 1 and 2:

| | |
|---|---|
| *1: | STR-100A-LP: Titanium dioxide (labeling name (TITANIUM DIOXIDE)) treated with hydrous silica (labeling name (HYDRATED SILICA)), aluminum hydroxide (labeling name (ALUMINUM HYDROXIDE)), and hydrogen dimethicone (labeling name (HYDROGEN DIMETHICONE)) |
| *2: | FINEX-30-LP: Zinc oxide (labeling name (ZINC OXIDE)) treated with hydrous silica (labeling name (HYDRATED SILICA)) and hydrogen dimethicone (labeling name (HYDROGEN DIMETHICONE)) |

As clear from the above results, the dispersions of Examples 1 to 10 containing a silicone oil, and the dispersions of Examples 11 to 15 containing an ester oil showed a low viscosity (less than 4,000 mPa·s) and further exhibited excellent stability over time. In contrast, the dispersions obtained in Comparative Examples 1 to 6 had a high viscosity (4,000 mPa·s or more) immediately after preparation, and became more viscous over time. As shown above, the carboxylic acid-modified silicones of the present invention can give low-viscosity dispersions having high dispersibility and better storage stability.

In the silicones of Comparative Structure 1 and Comparative Structure formula (26) in Comparative Examples, a carboxylic acid is bonded to a silicon atom via a hydrocarbon group. In contrast, the carboxylic acid-modified silicones of the present invention, by virtue of having a carboxyl group bonded to a silicon atom via an ester bond, can easily adsorb to the surface of powder particles and can prevent the particles from aggregation, thus allowing the powder to be dispersed with excellent dispersibility and stability.

### [Examples 16 and 17, and Comparative Examples 7 and 8]

Cosmetic W/O foundations were prepared according to the formulation described in Table 3.

### (Production method)

Components 1 to 7, 9, and 10 were mixed uniformly. Components 17 to 19 were mixed uniformly, and the resultant mixture was gently added to the mixture obtained above. The resultant mixture was stirred to give an emulsion. A mixture obtained beforehand by mixing components 8 and 13 to 16 by stirring, and component 12 were added. A foundation was thus obtained.

The cosmetics obtained were evaluated for (1) spreadability, (2) fitting to the skin, and (3) stability (over time) according to the following evaluation criteria.

### [Characteristics evaluation]

The cosmetics were evaluated by a panel of 10 professional members for spreadability and color tone according to the following evaluation criteria. The results of the 10 members were averaged. Each item was rated according to the judgement criteria below.

### [Evaluation criteria]

| | |
|---|---|
| 5 points: | very good |
| 4 points: | good |
| 3 points: | fair |
| 2 points: | slightly poor |
| 1 point: | poor. |

The average score was rated according to the following ∘× criteria.

### [Judgment criteria]

| | |
|---|---|
| ⊚: | Average score is 4.5 points or more. |
| ○: | Average score is 3.5 points or more and less than 4.5 points. |
| Δ: | Average score is 2.5 points or more and less than 3.5 points. |
| ×: | Average score is 1.5 points or more and less than 2.5 points. |
| ××: | Average score is less than 1.5 points. |

### [Stability (over time)]

The cosmetic was added into a 30 mL vial and was stored in a thermostatic chamber at 50°C for one week, and the viscosity was measured. The percentage change of the viscosity after the storage relative to the viscosity immediately after production (initial viscosity, temperature: 25°C) [(viscosity after storage - initial viscosity)/initial viscosity × 100] was determined.

| | |
|---|---|
| ⊚: | 0% or more and less than 25% |
| ○: | 25% or more and less than 40% |
| Δ: | 40% or more and less than 50% |
| ×: | 50% or more, or separation |

In this evaluation, the viscosity was measured at 25°C using a Brookfield viscometer (TVB-10, manufactured by Toki Sangyo Co., Ltd.). The initial viscosity was in the range of 5,000 to 200,000 mPa·s.

**[Table 3]**

| | Formulation (%) | Example 16 | Example 17 | Comp. Example 7 | Comp. Example 8 |
|---|---|---|---|---|---|
| 1 | Synthesis Example 1 (A) | 1.5 | - | - | - |
| 2 | Synthesis Example 2 (A) | - | 1.5 | - | - |
| 3 | Comparative structure 1 | - | - | 1.5 | - |
| 4 | Comparative structure 2 | - | - | - | 1.5 |
| 5 | Polyether-modified silicone (*1) | 1 | 1 | 1 | 1 |
| 6 | Crosslinked polyether-modified silicone (*2) | 3 | 3 | 3 | 3 |
| 7 | Dimethylpolysiloxane (Kinematic viscosity: 6 mm²/s) (B) | 3 | 3 | 3 | 3 |
| 8 | Dimethylpolysiloxane (Kinematic viscosity: 2 mm²/s) (B) | 9 | 9 | 9 | 9 |
| 9 | Glyceryl tri(caprylate/caprate) (B) | 5 | 5 | 5 | 5 |
| 10 | Neopentyl glycol dicaprate (B) | 2 | 2 | 2 | 2 |
| 11 | Polyglycerin-alkyl co-modified branched silicone (*3) | 2 | 2 | 2 | 2 |
| 12 | Spherical polymethyl silsesquioxane powder (*4) (C) | 1.5 | 1.5 | 1.5 | 1.5 |
| 13 | Hydrophobized titanium dioxide (*5) (C) | 6.9 | 6.9 | 6.9 | 6.9 |
| 14 | Hydrophobized red iron oxide (*6) (C) | 0.38 | 0.38 | 0.38 | 0.38 |
| 15 | Hydrophobized yellow iron oxide (*7) (C) | 0.6 | 0.6 | 0.6 | 0.6 |
| 16 | Hydrophobized black iron oxide (*8) (C) | 0.12 | 0.12 | 0.12 | 0.12 |
| 17 | Dipropylene glycol | 4.5 | 4.5 | 4.5 | 4.5 |
| 18 | Methyl p-hydroxybenzoate | 0.1 | 0.1 | 0.1 | 0.1 |
| 19 | Purified water | 58.9 | 58.9 | 58.9 | 58.9 |
| Total | | 100.0 | 100.0 | 100.0 | 100.0 |
| Spreadability | | ⊚ | ⊚ | △ | ○ |
| Fitting to the skin | | ⊚ | ⊚ | △ | △ |
| Stability (over time) | | ⊚ | ⊚ | × | △ |

| | | | | | |
|---|---|---|---|---|---|
| (*1) KSG-210 (INCI: DIMETHICONE/PEG-10/15 CROSSPOLYMER, DIMETHICONE) manufactured by Shin-Etsu Chemical Co., Ltd. (*2) KF-6017P (INCI: PEG-10 DIMETHICONE) manufactured by Shin-Etsu Chemical Co., Ltd. (*3) KF-6105 (INCI: LAURYL POLYGLYCERYL-3 POLYDIMETHYLSILOXYETHYL DIMETHICONE) manufactured by Shin-Etsu Chemical Co., Ltd. (*4) KMP-590 (INCI: POLYMETHYLSILSESQUIOXANE) manufactured by Shin-Etsu Chemical Co., Ltd. (*5) KTP-09W (INCI: TITANIUM DIOXIDE, ALUMINUM HYDROXIDE, TRIETHOXYSILYLETHYL POLYDIMETHYLSILOXYETHYL HEXYL DIMETHICONE) manufactured by Shin-Etsu Chemical Co., Ltd. (*6) KTP-09R (INCI: IRON OXIDES, TRIETHOXYSILYLETHYL POLYDIMETHYLSILOXYETHYL HEXYL DIMETHICONE) manufactured by Shin-Etsu Chemical Co., Ltd. (*7) KTP-09Y (INCI: IRON OXIDES, TRIETHOXYSILYLETHYL POLYDIMETHYLSILOXYETHYL HEXYL DIMETHICONE) manufactured by Shin-Etsu Chemical Co., Ltd. (*8) KTP-09B (INCI: IRON OXIDES, TRIETHOXYSILYLETHYL POLYDIMETHYLSILOXYETHYL HEXYL DIMETHICONE) manufactured by Shin-Etsu Chemical Co., Ltd. | | | | | |

### (Production method)

a: Components 5 to 7, 9 and 10 were mixed by stirring.
b: Components 1 to 4, 13 to 16, and 8 were mixed by stirring.
c: Components 17 to 19 were mixed uniformly.
d: The mixture obtained in c was added to the mixture obtained in a, and the resultant mixture was mixed by stirring to give an emulsion.
e: Component 12 and the mixture obtained in b were added to the emulsion obtained in d. The resultant mixture was mixed by stirring.

As clear from the above results, it has been demonstrated that the cosmetics of Examples 11 and 12 are highly stable while having good spreadability on the skin and well-fitting to the skin, as compared to the cosmetics of Comparative Examples 5 and 6.

Next, Examples will illustrate cosmetics containing the carboxylic acid-modified silicone of the present invention.

### [Example 18: Sunscreen milky lotion]

**[Table 4]**

| Formulation (%) | | |
|---|---|---|
| 1 | Crosslinked polyether-modified silicone (*1) | 3 |
| 2 | Synthesis Example 2 | 2 |
| 3 | Ethylhexyl methoxycinnamate | 7 |
| 4 | Dimethylpolysiloxane (Kinematic viscosity: 6 mm²/s) | 20 |
| 5 | Diphenylsiloxyphenyl trimethicone (*2) | 5 |
| 6 | Polyether-modified branched silicone (*3) | 1 |
| 7 | Titanium dioxide fine particles (*4) | 5 |
| 8 | Zinc oxide fine particles (*5) | 5 |
| 9 | 1,3-Butylene glycol | 5 |
| 10 | Sodium chloride | 0.5 |
| 11 | Sodium citrate | 0.2 |
| 12 | Purified water | 46.3 |
| Total | | 100.0 |

| | | |
|---|---|---|
| (*1) KSG-210 (INCI: DIMETHICONE/PEG-10/15 CROSSPOLYMER, DIMETHICONE) manufactured by Shin-Etsu Chemical Co., Ltd. (*2) KF-56A (INCI: DIPHENYL SILOXYPHENYL TRIMETHICONE) manufactured by Shin-Etsu Chemical Co., Ltd. (*3) KF-6028 (INCI: PEG-9 POLYDIMETHYLSILOXYETHYL DIMETHICONE) manufactured by Shin-Etsu Chemical Co., Ltd. (*4) MT-100TV manufactured by TAYCA Co., Ltd. (*5) MZX-508TS manufactured by TAYCA Co., Ltd. | | |

### (Production method)

Components 1 to 8 were thoroughly mixed using a homogenizing disperser to give a uniform mixture. Components 9 to 12 were mixed uniformly and then gently added to the mixture obtained above. The resultant mixture was stirred to give an emulsion as a sunscreen milky lotion. The sunscreen milky lotion obtained showed excellent usability with good spreadability and no stickiness.

### [Example 19: Makeup base]

**[Table 5]**

| Formulation (%) | | |
|---|---|---|
| 1 | Crosslinked polyether-modified silicone (*1) | 3 |
| 2 | Crosslinked dimethylpolysiloxane (*2) | 1 |
| 3 | Dimethylpolysiloxane (Kinematic viscosity: 6 mm²/s) | 8.4 |
| 4 | Polyether-modified silicone (*3) | 0.5 |
| 5 | Acrylic silicone resin solution (*4) | 2 |
| 6 | Spherical silicone composite powder (*5) | 2 |
| 7 | Dispersion of Example 2 | 20 |
| 8 | 1,3-Butylene glycol | 8 |
| 9 | Ethanol | 5 |
| 10 | Sodium chloride | 0.5 |
| 11 | Sodium citrate | 0.2 |
| 12 | Purified water | 49.4 |
| Total | | 100.0 |

| | | |
|---|---|---|
| (*1) KSG-210 (INCI: DIMETHICONE/PEG-10/15 CROSSPOLYMER, DIMETHICONE) manufactured by Shin-Etsu Chemical Co., Ltd. (*2) KSG-15 (INCI: DIMETHICONE/VINYL DIMETHICONE CROSSPOLYMER, CYCLOPENTASILOXANE) manufactured by Shin-Etsu Chemical Co., Ltd. (*3) KF-6017 (INCI: PEG-10 DIMETHICONE) manufactured by Shin-Etsu Chemical Co., Ltd. (*4) KP-545 (INCI: ACRYLATES/DIMETHICONE COPOLYMER, CYCLOPENTASILOXANE) manufactured by Shin-Etsu Chemical Co., Ltd. (*5) KSP-105 (INCI: VINYL DIMETHICONE/METHICONE SILSESQUIOXANE CROSSPOLYMER) manufactured by Shin-Etsu Chemical Co., Ltd. | | |

### (Production method)

Components 1 to 5 were thoroughly mixed to give a uniform mixture. Components 8 to 12 were mixed uniformly and then gently added to the mixture obtained above. The resultant mixture was stirred to give an emulsion. Components 6 and 7 were added. The resultant mixture was stirred uniformly to give a makeup base. The makeup base obtained showed excellent usability with good spreadability and no stickiness.

### [Example 20: Sunscreen cream]

**[Table 6]**

| Formulation (%) | | |
|---|---|---|
| 1 | Crosslinked polyether-modified silicone (*1) | 3 |
| 2 | Crosslinked dimethylpolysiloxane (*2) | 3 |
| 3 | Dimethylpolysiloxane (Kinematic viscosity: 6 mm²/s) | 3 |
| 4 | Decamethylpentasiloxane | 2 |
| 5 | Isononyl isononanoate | 4.8 |
| 6 | Polyether-modified silicone (*3) | 0.5 |
| 7 | Spherical silicone composite powder (*4) | 1 |
| 8 | Dispersion of Example 3 | 31.2 |
| 9 | Dispersion of Example 8 | 35 |
| 10 | 1,3-Butylene glycol | 8 |
| 11 | Sodium chloride | 0.5 |
| 12 | Sodium citrate | 0.2 |
| 13 | Purified water | 17.8 |
| Total | | 100.0 |

| | | |
|---|---|---|
| (*1) KSG-210 (INCI: DIMETHICONE/PEG-10/15 CROSSPOLYMER, DIMETHICONE) manufactured by Shin-Etsu Chemical Co., Ltd. (*2) KSG-15 (INCI: DIMETHICONE/VINYL DIMETHICONE CROSSPOLYMER, CYCLOPENTASILOXANE) manufactured by Shin-Etsu Chemical Co., Ltd. (*3) KF-6028P (INCI: PEG-9 POLYDIMETHYLSILOXYETHYL DIMETHICONE) manufactured by Shin-Etsu Chemical Co., Ltd. (*4) KSP-100 (INCI: VINYL DIMETHICONE/METHICONE SILSESQUIOXANE CROSSPOLYMER) manufactured by Shin-Etsu Chemical Co., Ltd. | | |

### (Production method)

Components 1 to 7 were thoroughly mixed to give a uniform mixture. Components 10 to 13 were mixed uniformly and then gently added to the mixture obtained above. The resultant mixture was stirred to give an emulsion. Components 8 and 9 were added. The resultant mixture was stirred uniformly to give a sunscreen cream. The sunscreen cream obtained exhibited good spreadability and no stickiness, thus being shown to have excellent usability, and was also excellent in stability.

### [Example 21: Sunscreen cream]

**[Table 7]**

| Formulation (%) | | |
|---|---|---|
| 1 | Crosslinked polyether-modified silicone (*1) | 2 |
| 2 | Crosslinked phenyl-modified silicone (*2) | 2 |
| 3 | Polyether-alkyl co-modified branched silicone (*3) | 1.5 |
| 4 | Dimethylpolysiloxane (Kinematic viscosity: 2 mm²/s) | 23.5 |
| 5 | (C13-15) alkane | 3 |
| 6 | Distearyl ammonium hectorite | 0.8 |
| 7 | Ethylhexyl methoxycinnamate | 3 |
| 8 | Ethylhexyl salicylate | 2 |
| 9 | Bis-ethylhexyloxyphenol methoxyphenyl triazine | 2 |
| 10 | Diphenylsiloxyphenyl trimethicone (*4) | 5 |
| 11 | Phenyl-modified spherical silicone composite powder (*5) | 0.5 |
| 12 | Dispersion of Example 13 | 12 |
| 13 | 1,3-Butylene glycol | 3 |
| 14 | Ethanol | 5 |
| 15 | Sodium chloride | 0.5 |
| 16 | Sodium citrate | 0.2 |
| 17 | Purified water | 34 |
| Total | | 100.0 |

| | | |
|---|---|---|
| (*1) KSG-240 (INCI: DIMETHICONE/PEG-10/15 CROSSPOLYMER, CYCLOPENTASILOXANE) manufactured by Shin-Etsu Chemical Co., Ltd. (*2) KSG-18A (INCI: DIMETHICONE/PHENYL VINYL DIMETHICONE CROSSPOLYMER, DIPHENYL SILOXYPHENYL TRIMETHICONE) manufactured by Shin-Etsu Chemical Co., Ltd. (*3) KF-6038 (INCI: LAURYL PEG-9 POLYDIMETHYLSILOXYETHYL DIMETHICONE) manufactured by Shin-Etsu Chemical Co., Ltd. (*4) KF-56A (INCI: DIPHENYL SILOXYPHENYL TRIMETHICONE) manufactured by Shin-Etsu Chemical Co., Ltd. (*5) KSP-300 (INCI: DIPHENYL DIMETHICONE/VINYL DIPHENYL DIMETHICONE/SILSESQUIOXANE CROSSPOLYMER) manufactured by Shin-Etsu Chemical Co., Ltd. | | |

### (Production method)

Components 1 to 11 were thoroughly mixed to give a uniform mixture. Components 13 to 17 were mixed uniformly and then gently added to the mixture obtained above. The resultant mixture was stirred to give an emulsion. Component 12 was added. The resultant mixture was stirred uniformly to give a sunscreen cream. The sunscreen cream obtained exhibited good spreadability and no stickiness, thus being shown to have excellent usability, and was also excellent in stability.

### [Example 22: Sunscreen cream]

**[Table 8]**

| Formulation (%) | | |
|---|---|---|
| 1 | Crosslinked polyglycerin-alkyl co-modified silicone (*1) | 2 |
| 2 | Crosslinked alkyl-modified silicone (*2) | 2 |
| 3 | Polyglycerin-alkyl co-modified branched silicone (*3) | 4 |
| 4 | Neopentylglycol dicaprate | 8 |
| 5 | Isotridecyl isononanoate | 13 |
| 6 | Diphenylsiloxyphenyl trimethicone (*4) | 5 |
| 7 | Alkyl-modified spherical silicone composite powder (*5) | 2 |
| 8 | Polymethylsilsesquioxane (*6) | 0.5 |
| 9 | Dispersion of Example 14 | 12 |
| 10 | Ethylhexyl glycerin | 3 |
| 11 | Sorbitol | 0.2 |
| 12 | Ethanol | 0.5 |
| 13 | Sodium citrate | 0.2 |
| 14 | Sodium chloride | 0.5 |
| 15 | Hydroxyethyl cellulose | 0.3 |
| 16 | Arbutin | 0.5 |
| 17 | Allantoin | 0.1 |
| 18 | Phenoxyethanol | 0.3 |
| 19 | Methylparaben | 0.1 |
| 20 | Purified water | 45.8 |
| Total | | 100.0 |

| | | |
|---|---|---|
| (*1) KSG-820 (INCI: LAURYL DIMETHICONE/POLYGLYCERYL-3 CROSSPOLYMER, ISODODECANE) manufactured by Shin-Etsu Chemical Co., Ltd. (*2) KSG-44 (INCI: VINYL DIMETHICONE/LAURYL DIMETHICONE CROSSPOLYMER, SQUALANE) manufactured by Shin-Etsu Chemical Co., Ltd. (*3) KF-6105 (INCI: LAURYL POLYGLYCERYL-3 POLYDIMETHYLSILOXYETHYL DIMETHICONE) manufactured by Shin-Etsu Chemical Co., Ltd. (*4) KF-56A (INCI: DIPHENYL SILOXYPHENYL TRIMETHICONE) manufactured by Shin-Etsu Chemical Co., Ltd. (*5) KSP-441 (INCI: POLYSILICONE-22) manufactured by Shin-Etsu Chemical Co., Ltd. (*6) KSP-591 (INCI: POLYMETHYLSILSESQUIOXANE) manufactured by Shin-Etsu Chemical Co., Ltd. | | |

### (Production method)

Components 1 to 8 were thoroughly mixed to give a uniform mixture. Components 10 to 20 were mixed uniformly and then gently added to the mixture obtained above. The resultant mixture was stirred to give an emulsion. Component 9 was added. The resultant mixture was stirred uniformly to give a sunscreen cream. The sunscreen cream obtained exhibited good spreadability and no stickiness, thus being shown to have excellent usability, and was also excellent in stability.

### [Example 23: Foundation]

**[Table 9]**

| Formulation (%) | | |
|---|---|---|
| 1 | Crosslinked polyether-modified silicone (*1) | 3 |
| 2 | Crosslinked phenyl-modified silicone (*2) | 3 |
| 3 | Dimethylpolysiloxane (Kinematic viscosity: 6 mm²/s) | 5 |
| 4 | Polyether-alkyl co-modified branched silicone (*3) | 1.5 |
| 5 | Ethylhexyl methoxycinnamate | 2 |
| 6 | Ethylhexyl salicylate | 2 |
| 7 | Octocrylene | 2 |
| 8 | Diphenylsiloxyphenyl trimethicone (*4) | 7 |
| 9 | Synthesis Example 3 | 3 |
| 10 | Hydrophobized titanium dioxide (*5) | 17 |
| 11 | Hydrophobized red iron oxide (*6) | 1 |
| 12 | Hydrophobized yellow iron oxide (*7) | 1.8 |
| 13 | Hydrophobized black iron oxide (*8) | 0.2 |
| 14 | Glyceryl tri(caprylate/caprate) | 5 |
| 15 | 1,3-Butylene glycol | 2 |
| 16 | Sodium chloride | 0.5 |
| 17 | Sodium citrate | 0.2 |
| 18 | Purified water | 43.8 |
| Total | | 100.0 |

| | | |
|---|---|---|
| (*1) KSG-210 (INCI: DIMETHICONE/PEG-10/15 CROSSPOLYMER, DIMETHICONE) manufactured by Shin-Etsu Chemical Co., Ltd. (*2) KSG-18A (INCI: DIMETHICONE/PHENYL VINYL DIMETHICONE CROSSPOLYMER, DIPHENYL SILOXYPHENYL TRIMETHICONE) manufactured by Shin-Etsu Chemical Co., Ltd. (*3) KF-6038 (INCI: LAURYL PEG-9 POLYDIMETHYLSILOXYETHYL DIMETHICONE) manufactured by Shin-Etsu Chemical Co., Ltd. (*4) KF-56A (INCI: DIPHENYL SILOXYPHENYL TRIMETHICONE) manufactured by Shin-Etsu Chemical Co., Ltd. (*5) Titanium dioxide (INCI: TITANIUM DIOXIDE) treated with KF-9901 (INCI: HYDROGEN DIMETHICONE), manufactured by Shin-Etsu Chemical Co., Ltd. (*6) Red iron oxide (INCI: IRON OXIDES) treated with KF-9901 (INCI: HYDROGEN DIMETHICONE), manufactured by Shin-Etsu Chemical Co., Ltd. (*7) Yellow iron oxide (INCI: IRON OXIDES) treated with KF-9901 (INCI: HYDROGEN DIMETHICONE), manufactured by Shin-Etsu Chemical Co., Ltd. (*8) Black iron oxide (INCI: IRON OXIDES) treated with KF-9901 (INCI: HYDROGEN DIMETHICONE), manufactured by Shin-Etsu Chemical Co., Ltd. | | |

### (Production method)

Components 1 to 8 were thoroughly mixed to give a uniform mixture.
Components 15 to 18 were mixed uniformly and then gently added to the mixture obtained above. The resultant mixture was stirred to give an emulsion. Component 9 was added. The resultant mixture was stirred uniformly to give a foundation. The foundation obtained exhibited good spreadability and no stickiness, thus being shown to have excellent usability, and was also excellent in stability.

### (Production method)

a: Components 1 to 8 were mixed by stirring.
b: Components 9 to 14 were mixed by stirring.
c: Components 15 to 18 were mixed uniformly.
d: The mixture obtained in c was added to the mixture obtained in a. The resultant mixture was mixed by stirring to give an emulsion.
e: The mixture obtained in b was added to the emulsion obtained in d. The resultant mixture was mixed by stirring.

The foundation obtained showed excellent usability with good spreadability and no stickiness.

### [Example 24: Foundation]

**[Table 10]**

| Formulation (%) | | |
|---|---|---|
| 1 | Crosslinked polyglycerin-alkyl co-modified silicone (*1) | 3 |
| 2 | Crosslinked alkyl-modified silicone (*2) | 5 |
| 3 | Polyglycerin-alkyl co-modified branched silicone (*3) | 3 |
| 4 | Ethylhexyl palmitate | 3 |
| 5 | Dispersion of Example 4 | 20 |
| 6 | Dispersion of Example 9 | 15 |
| 7 | Synthesis Example 4 | 0.3 |
| 8 | Hydrophobized titanium dioxide (*4) | 8.5 |
| 9 | Hydrophobized red iron oxide (*5) | 0.5 |
| 10 | Hydrophobized yellow iron oxide (*6) | 0.9 |
| 11 | Hydrophobized black iron oxide (*7) | 0.1 |
| 12 | Isotridecyl isononanoate | 1.7 |
| 13 | 1,3-Butylene glycol | 5 |
| 14 | Sodium chloride | 0.5 |
| 15 | Sodium citrate | 0.2 |
| 16 | Purified water | 33.3 |
| Total | | 100.0 |

| | | |
|---|---|---|
| (*1) KSG-820 (INCI: LAURYL DIMETHICONE/POLYGLYCERYL-3 CROSSPOLYMER, ISODODECANE) manufactured by Shin-Etsu Chemical Co., Ltd. (*2) KSG-44 (INCI: VINYL DIMETHICONE/LAURYL DIMETHICONE CROSSPOLYMER, SQUALANE) manufactured by Shin-Etsu Chemical Co., Ltd. (*3) KF-6105 (INCI: LAURYL POLYGLYCERYL-3 POLYDIMETHYLSILOXYETHYL DIMETHICONE) manufactured by Shin-Etsu Chemical Co., Ltd. (*4) KTP-09W (INCI: TITANIUM DIOXIDE, ALUMINUM HYDROXIDE, TRIETHOXYSILYLETHYL POLYDIMETHYLSILOXYETHYL HEXYL DIMETHICONE) manufactured by Shin-Etsu Chemical Co., Ltd. (*5) KTP-09R (INCI: IRON OXIDES, TRIETHOXYSILYLETHYL POLYDIMETHYLSILOXYETHYL HEXYL DIMETHICONE) manufactured by Shin-Etsu Chemical Co., Ltd. (*6) KTP-09Y (INCI: IRON OXIDES, TRIETHOXYSILYLETHYL POLYDIMETHYLSILOXYETHYL HEXYL DIMETHICONE) manufactured by Shin-Etsu Chemical Co., Ltd. (*7) KTP-09B (INCI: IRON OXIDES, TRIETHOXYSILYLETHYL POLYDIMETHYLSILOXYETHYL HEXYL DIMETHICONE) manufactured by Shin-Etsu Chemical Co., Ltd. | | |

### (Production method)

a: Components 1 to 4 were mixed by stirring.
b: Components 7 to 12 were mixed by stirring.
c: Components 13 to 16 were mixed uniformly.
d: The mixture obtained in c was added to the mixture obtained in a. The resultant mixture was mixed by stirring to give an emulsion.
e: The mixture obtained in b, and components 5 and 6 were added to the mixture obtained in d. The resultant mixture was mixed by stirring.

The foundation obtained showed excellent usability with good spreadability and no stickiness.

### [Example 25: Sunscreen cream]

**[Table 11]**

| Formulation (%) | | |
|---|---|---|
| 1 | Dispersion of Example 3 | 10 |
| 2 | Dispersion of Example 8 | 10 |
| 3 | Dimethylpolysiloxane (Kinematic viscosity: 6 mm²/s) | 2 |
| 4 | Diphenylsiloxyphenyl trimethicone (*4) | 0.5 |
| 5 | Ethylhexyl methoxycinnamate | 0.5 |
| 6 | (Ammonium acryloyldimethyltaurate/vinyl pyrrolidone) copolymer | 2 |
| 7 | (Acrylamide/sodium acryloyldimethyltaurate) copolymer | 0.4 |
| 10 | Polyglyceryl-10 myristate | 4 |
| 11 | Polyglyceryl-10 dimyristate | 3 |
| 12 | 1,3-Butylene glycol | 0.2 |
| 13 | Ethanol | 4 |
| 14 | Citric acid | 0.1 |
| 15 | Phenoxyethanol | 0.2 |
| 16 | Purified water | 63.1 |
| Total | | 100.0 |

### (Production method)

a: Components 6 to 16 were mixed uniformly.
b: Components 1 to 5 were mixed uniformly.
c: The mixture obtained in b was added to the mixture obtained in a. The resultant mixture was mixed by stirring to give an emulsion.

The sunscreen cream obtained spread well and was smooth and non-sticky, thus being shown to have excellent usability.

### [Example 26: Lipstick]

**[Table 12]**

| Formulation (%) | | |
|---|---|---|
| 1 | Candelilla wax | 8 |
| 2 | Polyethylene wax | 5 |
| 3 | Microcrystalline wax | 1 |
| 4 | Long-chain alkyl-containing acrylic silicone resin (*1) | 10 |
| 5 | Macadamia nut oil | 6 |
| 6 | Diisostearyl malate | 1 |
| 7 | Isotridecyl isononanoate | 4 |
| 8 | Acrylic silicone resin solution (*2) | 45 |
| 9 | Polyglyceryl triisostearate | 5.4 |
| 10 | Synthesis Example 3 | 3 |
| 11 | Sericite | 1 |
| 12 | Red #201 | 0.2 |
| 13 | Red #202 | 0.5 |
| 14 | Yellow #4 aluminum lake | 1.6 |
| 15 | Red iron oxide | 1 |
| 16 | Black iron oxide | 0.3 |
| 17 | Titanium dioxide | 4 |
| 18 | Mica | 5 |
| Total | | 100.0 |

| | | |
|---|---|---|
| (*1) KP-561P (INCI: ACRYLATES/STEARYL ACRYLATE/DIMETHICONE COPOLYMER) manufactured by Shin-Etsu Chemical Co., Ltd. (*2) KP-550 (INCI: ACRYLATES/DIMETHICONE COPOLYMER, ISODODECANE) manufactured by Shin-Etsu Chemical Co., Ltd. | | |

### (Production method)

a: Components 1 to 7 were mixed while performing heating.
b: Components 9 to 18 were mixed uniformly.
c: Component 8 was added to the mixture obtained in b. The resultant mixture was mixed.

The lipstick obtained spread well, was smooth and well-fitting to the skin, had good shine, and showed excellent cosmetic durability.

### [Example 27: Eyeliner]

**[Table 13]**

| Formulation (%) | | |
|---|---|---|
| 1 | Decamethylpentasiloxane | 6 |
| 2 | Dimethylpolysiloxane (Kinematic viscosity: 6 mm²/s) | 5 |
| 3 | Jojoba oil | 2 |
| 4 | Polyether-modified silicone (*1) | 1 |
| 5 | Polyether-alkyl co-modified branched silicone (*2) | 1 |
| 6 | Silicone resin solution (*3) | 15 |
| 7 | Synthesis Example 5 | 1.5 |
| 8 | Black iron oxide (*4) | 20 |
| 9 | Ethanol | 5 |
| 10 | Sodium chloride | 0.5 |
| 11 | Sodium citrate | 0.2 |
| 12 | Purified water | 42.8 |
| Total | | 100.0 |

| | | |
|---|---|---|
| (*1) KF-6017 (INCI: PEG-10 DIMETHICONE) manufactured by Shin-Etsu Chemical Co., Ltd. (*2) KF-6038 (INCI: LAURYL PEG-9 POLYDIMETHYLSILOXYETHYL DIMETHICONE) manufactured by Shin-Etsu Chemical Co., Ltd. (*3) KF-9021 (INCI: TRIMETHYLSILOXYSILICATE, CYCLOPENTASILOXANE) manufactured by Shin-Etsu Chemical Co., Ltd. (*4) KTP-09B (INCI: IRON OXIDES, TRIETHOXYSILYLETHYL POLYDIMETHYLSILOXYETHYL HEXYL DIMETHICONE) manufactured by Shin-Etsu Chemical Co., Ltd. | | |

### (Production method)

a: Components 1 to 8 were mixed.
b: Components 9 to 12 were mixed uniformly.
c: The mixture obtained in b was added to the mixture obtained in a. The resultant mixture was emulsified.

The eyeliner obtained spread well, was smooth and well-fitting to the skin, and showed excellent water resistance and usability.

### [Example 28: Sunscreen cream (O/W)]

**[Table 14]**

| Formulation (%) | | |
|---|---|---|
| 1 | Polyether alkyl-modified silicone (*1) | 2 |
| 2 | 1,3-Butylene glycol | 10 |
| 3 | Crosslinked alkyl-modified silicone (*2) | 2 |
| 4 | Squalane | 17.7 |
| 5 | Isostearic acid | 0.3 |
| 6 | Synthesis Example 4 | 0.2 |
| 7 | Titanium dioxide fine particles (*3) | 3 |
| 8 | Zinc oxide fine particles (*4) | 5 |
| 9 | Isotridecyl isononanoate | 6 |
| 10 | Sodium hydroxide | 0.042 |
| 11 | Tinosorb M (*5) | 5 |
| 12 | (Acrylamide/sodium acryloyldimethyltaurate) copolymer | 1 |
| 13 | Purified water | 47.758 |
| Total | | 100.0 |

| | | |
|---|---|---|
| (*1) Lauryl group-PEG-9 co-modified silicone, HLB = 10.5 (*2) KSG-44 (INCI: VINYL DIMETHICONE/LAURYL DIMETHICONE CROSSPOLYMER, SQUALANE) manufactured by Shin-Etsu Chemical Co., Ltd. (*3) STR-100W-OTS manufactured by SAKAI CHEMICAL INDUSTRY CO., LTD. (*4) MZX-508OTS manufactured by TAYCA Co., Ltd. (*5) Manufactured by BASF | | |

### (Production method)

a: Components 6 to 9 were mixed.
b: Components 3 to 5 were added to components 1 and 2 to give a gel.
c: The mixture obtained in a was added to the gel obtained in b. The resultant mixture was mixed by stirring.
d: Components 10 to 13 were added to the mixture obtained in c. The resultant mixture was emulsified.

The sunscreen cream obtained spread well and was dewy, smooth, and non-sticky, thus being shown to have excellent usability.

### [Example 29: Sunscreen cream (O/W)]

**[Table 15]**

| Formulation (%) | | |
|---|---|---|
| 1 | Polyether alkyl-modified silicone (*1) | 2 |
| 2 | 1,3-Butylene glycol | 10 |
| 3 | Water | 2 |
| 4 | Squalane | 17.7 |
| 5 | Isostearic acid | 0.3 |
| 6 | Synthesis Example 5 | 0.2 |
| 7 | Zinc oxide fine particles (*2) | 8 |
| 8 | Isotridecyl isononanoate | 4 |
| 9 | Polyether-modified silicone (*3) | 4.2 |
| 10 | Titanium dioxide fine particles (*4) | 6 |
| 11 | Sodium hydroxide | 0.042 |
| 12 | (Acrylamide/sodium acryloyldimethyltaurate) copolymer | 1 |
| 13 | Purified water | 44.558 |
| Total | | 100.0 |

| | | |
|---|---|---|
| (*1) Lauryl group-PEG-9 co-modified silicone, HLB = 10.5 (*2) MZX-508OTS manufactured by TAYCA Co., Ltd. (*3) KF-6013 (INCI: PEG-9 DIMETHICONE) manufactured by Shin-Etsu Chemical Co., Ltd. (*4) STR-100W-OTS manufactured by SAKAI CHEMICAL INDUSTRY CO., LTD. | | |

### (Production method)

a: Components 6 to 8 were mixed.
b: A portion of component 13 was added to components 9 and 10. The resultant mixture was mixed.
c: Components 4 and 5 were added to components 1 to 3 to give a gel.
d: The mixture obtained in a was added to the gel obtained in c. The resultant mixture was mixed by stirring.
e: Components 11 and 12, and the remainder of component 13 were added to the mixture obtained in d. The resultant mixture was emulsified.
f: The emulsion obtained in e and the mixture obtained in b were mixed.

The sunscreen cream obtained spread well and was dewy, smooth, and non-sticky, thus being shown to have excellent usability.

### [Example 30: Foundation (O/W)]

**[Table 16]**

| Formulation (%) | | |
|---|---|---|
| 1 | Polyether alkyl-modified silicone (*1) | 2 |
| 2 | 1,3-Butylene glycol | 10 |
| 3 | Water | 2 |
| 4 | Squalane | 17.7 |
| 5 | Isostearic acid | 0.3 |
| 6 | Synthesis Example 3 | 0.1 |
| 7 | Red iron oxide (*2) | 0.4 |
| 8 | Yellow iron oxide (*3) | 0.9 |
| 9 | Black iron oxide (*4) | 0.1 |
| 10 | Isotridecyl isononanoate | 3 |
| 11 | Polyether-modified silicone (*5) | 4.2 |
| 12 | Titanium dioxide (*6) | 7.5 |
| 13 | Sodium hydroxide | 0.092 |
| 14 | Carbomer | 0.2 |
| 15 | Xanthan gum (2% aqueous solution) | 2 |
| 16 | Purified water | 49.508 |
| Total | | 100.0 |

| | | |
|---|---|---|
| (*1) Lauryl group-PEG-9 co-modified silicone, HLB = 10.5 (*2) ALT-RHP-10 manufactured by Miyoshi Kasei, Inc. (*3) ALT-YHP-10 manufactured by Miyoshi Kasei, Inc. (*4) ALT-BHP-10 manufactured by Miyoshi Kasei, Inc. (*5) KF-6013 (INCI: PEG-9 DIMETHICONE) manufactured by Shin-Etsu Chemical Co., Ltd. (*6) ALT-TSR-10 manufactured by Miyoshi Kasei, Inc. | | |

### (Production method)

a: Components 6 to 10 were mixed.
b: A portion of component 16 was added to components 11 and 12. The resultant mixture was mixed.
c: Components 4 and 5 were added to components 1 to 3 to form a gel.
d: The mixture obtained in a was added to the gel obtained in c. The resultant mixture was mixed by stirring.
e: Components 13 to 15, and the remainder of component 16 were added to the mixture obtained in d. The resultant mixture was emulsified.
f: The mixture obtained in b was added to the emulsion obtained in e.

The foundation obtained spread well and was dewy, smooth, and non-sticky, thus being shown to have excellent usability.

### [Example 31: Shaking sunscreen]

**[Table 17]**

| Formulation (%) | | |
|---|---|---|
| 1 | Zinc oxide fine particles (*1) | 16 |
| 2 | Synthesis Example 2 | 0.2 |
| 3 | Isododecane | 5 |
| 4 | Alkyl (C12-16) benzoate | 7.3 |
| 5 | Ethylhexyl methoxycinnamate | 7.5 |
| 6 | Diethylamino hydroxybenzoyl hexyl benzoate | 1 |
| 7 | Bis-ethylhexyloxyphenol methoxyphenyl triazine | 2 |
| 8 | Decamethylcyclopentasiloxane | 15 |
| 9 | Silica | 1 |
| 10 | Spherical silicone composite powder (*2) | 3 |
| 11 | Sodium alginate | 2 |
| 12 | Ethanol | 5 |
| 13 | 1,3-Butylene glycol | 5 |
| 14 | Purified water | 30 |
| Total | | 100.0 |

| | | |
|---|---|---|
| (*1) MZX-508OTS manufactured by TAYCA Co., Ltd. (*2) KSP-100 (INCI: VINYL DIMETHICONE/METHICONE SILSESQUIOXANE CROSSPOLYMER) manufactured by Shin-Etsu Chemical Co., Ltd. | | |

### (Production method)

a: Components 1 to 3 were mixed.
b: Components 4 to 10 were added to the mixture obtained in a. The resultant mixture was mixed.
c: Components 11 to 14 were added to the mixture obtained in b. The resultant mixture was mixed by stirring.

The sunscreen obtained spread well and was dewy, smooth, and non-sticky, thus being shown to have excellent usability.

From the foregoing, it has been shown that the cosmetics containing the carboxylic acid-modified silicone of the present invention attain excellent powder dispersibility and dispersion stability, have good spreadability, are well-fitting to the skin, and exhibit excellent stability over time.

The present invention is not limited to the aforementioned embodiments. The embodiments discussed above are only illustrative, and any embodiments having substantially the same configuration as the technical concept set forth in the claims of the present invention and exerting equivalent functions and effects fall within the technical scope of the present invention.

## Claims

1. A cosmetic comprising:
(A) 0.1 to 10% by mass of a carboxylic acid-modified silicone with a weight average molecular weight of 1,000 to 5,000 represented by formula (1) below:
[Chem. 1]
[R¹₂SiO_{2/2}]ₐ[R¹R²SiO_{2/2}]_{b}[R¹R³SiO_{2/2}]_{c}[R¹SiO_{3/2}]_{d}[SiO_{4/2}]ₑ[R¹₃SiO_{1/2}]_{f}[R¹R²SiO_{1/2}]_{g} (1)
wherein R¹ independently at each occurrence is a monovalent organic group selected from C₁-C₂₀ alkyl groups, C₆-C₁₅ aryl groups, C₇-C₁₅ aralkyl groups, and -O-R⁴ wherein Q is a C₂-C₄ alkylene group, and R⁴ is a C₃-C₃₀ alkyl group, R² independently at each occurrence is a carboxylic acid group represented by formula (2) below: 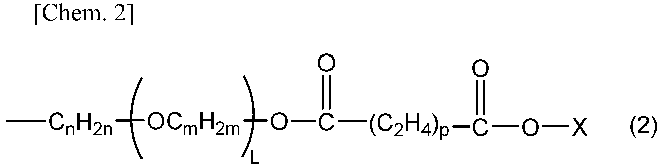 wherein X denotes a hydrogen atom or a monovalent cation, n is 2 to 4, m is 2 to 4, L is 0 to 20, and p is 2 to 6,
R³ is a group represented by formula (3) or (4) below: 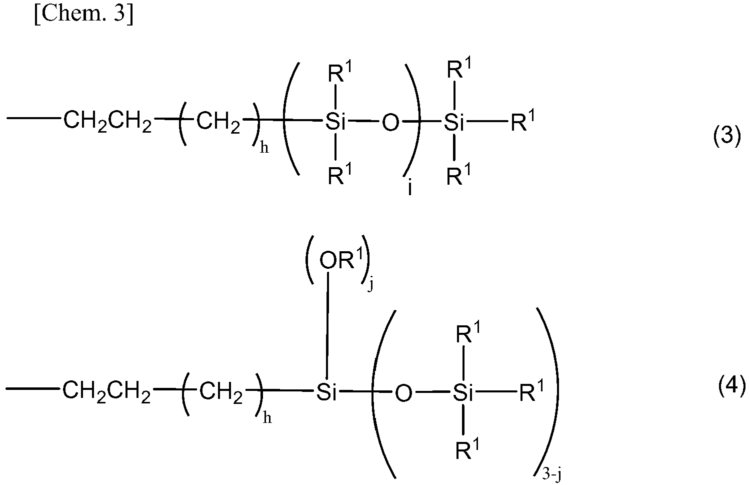 wherein R¹ independently at each occurrence is the same as above, h is 0 to 5, i is 0 to 80, and j is 0 to 3,
a is 0 to 20, b is 0 to 5, c is 0 to 3, d is 0 or greater, e is 0 or greater, f and g are numbers satisfying f + g = 2 + d + e × 2, and b and g are not 0 at the same time;
(B) 1 to 90% by mass of an oil; and
(C) 2 to 95% by mass of a powder.

2. The cosmetic according to claim 1, further comprising (D) 0.1 to 30% by mass of an organic UV absorber.

3. The cosmetic according to claim 1, wherein the oil (B) is one or more selected from silicone oils, hydrocarbon oils, ester oils, glyceride oils, natural animal and plant oils and fats, semi-synthetic oils and fats, and higher alcohols.

4. The cosmetic according to claim 1, wherein the powder (C) is one or more selected from titanium dioxide, zinc oxide, and colored pigments.

5. The cosmetic according to any one of claims 1 to 4, wherein the cosmetic further comprises a surfactant (E) and water, and is an emulsion.
